# EUROPEAN PATENT SPECIFICATION

(11) **EP 2 106 704 B1**
(45) Date of publication and mention of the grant of the patent: **25.12.2013**
(21) Application number: 09155916.1
(22) Date of filing: 23.03.2009
(51) Int. Cl.: A23L 1/22, A61K 8/11, C11D 3/50, A61K 8/24, A61Q 13/00, A61Q 15/00, C11D 3/06, C11D 3/22, C11D 7/36, A61K 8/73, A23L 2/39

(54) **Particles having a high load of fragrance or flavor oil**
Partikel mit einer hohen Duftstoff- oder Duftöllast
Particules disposant d'une quantité élevée de fragrance ou d'huile aromatique

(30) Priority: 02.04.2008 US 61174
(43) Date of publication of application: 07.10.2009
(73) Proprietor: Symrise AG, 37603 Holzminden (DE)
(72) Inventor: Mushock, Steven, HIGHLANDS, NJ 07732 (US); McDermott, Keith, BOUND BROOK, NJ 08805 (US); Clothier, James G., RIDGEWOOD, NJ 07450 (US)
(74) Representative: Eisenführ, Speiser & Partner

(56) References cited:
- EP-A- 0 550 067
- US-A- 4 209 417
- US-A1- 2005 272 628
- US-A1- 2007 155 649
- US-B1- 6 608 017

## Description

### Field of the invention:

The present invention relates to (preferably spray-dried) particles having a high load of fragrance (perfume) or flavor oil, a process for their preparation, its use for perfuming and/or flavoring products and products comprising such particles.

### Background of the invention:

The perfuming or flavoring of products has been known for a long time. For perfuming or flavoring purposes either liquids or solids (i.e. fragrances or flavors applied to solid carriers, e.g. spray-dried, or enclosed in solid carriers) are conventionally used in this procedure.

US 6,790,814 teaches a three part core-shell system consisting of a core, an intermediate coating and an external coating. Phosphate is listed as one of several possible external coating components.

US 4,209,417 discloses perfumed particles consisting of from 30 wt.-% to 70 wt.-% water-insoluble perfume oil, from 25% to 65% of a water-soluble polymer which will dissolve in water at a temperature of less than 100° C, and an effective amount of an emulsifier. Preferably polyvinyl alcohol is used as the water-soluble polymer.

US 6,608,017 discloses anencapsulated oil particles comprising a) starch which has been modified by treatment with octenylsuccinic acid anhydride; b) oil; and c) hydrophobically modified silica; wherein the amount of hydrophobic silica is less than or equal to 10% by weight of said oil. The amount of encapsulated oil may reach 48 wt.-%.

US 2005/272628 discloses a process for making an encapsulated ingredient comprising (a) preparing a mixture comprising starch, water, acid and an ingredient for encapsulation, the acid being incorporated in the mixture in an amount sufficient to lower the pH of the starch-water mixture by at least 0.25 units; and (b) atomising and drying the mixture thereby forming encapsulated ingredient. The starch is preferably octenyl-succuinate-modified starch. The encapsulated ingredient may comprise at least 60 wt % of an ingredient, e.g. a perfume oil.

US 2007/155649 discloses a dry powder comprising dry perfuming or flavoring microcapsules and a fireproofing agent in an amount sufficient to provide an St-1 classification to the powder to reduce its dust hazard explosive class, but below that which would deleteriously affect the perfuming or flavoring properties of the perfuming or flavoring ingredients or of products to which the microcapsules are incorporated. The fireproofing agent may be incorporated in the microcapsules. The fireproofing agent is selected from the group consisting of sodium silicate, potassium silicate, monoammonium phosphate or carbonate, diammonium phosphate, mono-, di- or trisodium phosphate, sodium hypophosphite, melamine cyanurate, and mixtures thereof.

EP 0 550 067 discloses a method for encapsulating oils such as fragrance or flavoring oils in a water-sensitive cellular solid matrix, comprising drying an aqueous emulsion containing the oil to be encapsulated, a non-crosslinked lipophilically modified starch that undergoes cross-linking under the drying conditions, and a polyhydroxy compound that forms with the polysaccharide material a continuous aqueous phase in which oil is dispersible as a discontinuous phase.

WO 98/042818 describes a core particle with a porous carrier and a glassy encapsulating material surrounded by one or two additional coatings. Phosphate is listed as one of several possible additives to the external coating.

EP 1 160 311 discloses a glassy encapsulation material that can additionally contain porous additives and additional coatings around the core material. Phosphate is listed as one of several possible additives to the core material and coating.

Glassy particles are conventionally formed by dissolving the components into a syrup, allowing it to cool below the glass transition temperature, then using mechanical means to break the glass into particles.

Encapsulation of fragrance or flavor oil by means of spray-drying, as described, for example, in US 3,159,585, US 3,971,852, US 4,532,145 or US 5,124,162, is well known in the art. The content of fragrance or flavor oil in known spray-dried particles comprising is conventionally achievable up to a content of 40-45 wt.% fragrance or flavor oil, based on the total weight of the particles.

The encapsulation of fragrances is well known in the art. An antiperspirant/deodorant containing microcapsules is disclosed in US 5,176,903 where a fragrance oil and ester are encapsulated by a food starch and polysaccharide composition.

Such particles can be used for perfuming or flavoring the following examples of consumer products, such as underarm deodorants and antiperspirants, solid sticks and aerosols, powdered laundry detergents, fragranced cat litter, and the like.

The consumer product market is a very competitive market, where the price of the product must also be competitive. Accordingly, it is desired to lower the production costs, in particular the raw material costs of the perfuming and flavoring particles used for perfuming or flavoring the perfumed or flavored products. One way to lower the raw material costs is to raise the content of the fragrance and/or flavor oil per particle so that the amount of the perfumed or flavored particles used to the perfumed or flavored consumer products can be reduced.

Further to this it is often desired to control, in particular to retard the release of the perfume or flavor out of the consumer products, in particular when the perfumed or flavored consumer products will be used in (cold) water.

Moreover, it is desired that the fragrance or flavor oil containing particles as raw material can be easily prepared and are stable both mechanically and against oxidation.

### Summary of the invention:

Thus, the main objects of the present invention relate to the provision of improved solid fragrance (perfume) or flavor oil containing particles
- comprising a high(er) content of fragrance and/or flavor oil to lower the production costs,
- comprising a controlled release of the fragrance and/or flavor oil out of the solid particles,
- and/or comprising a high(er) (oxidation) stability and/or
- are easy to prepare and/or to handle in further manufacturing processes of perfumed or flavored consumer products.

One or more of the main objects are solved by the subject matter of the independent claims. Preferred embodiments are set out in the dependent claims and are also disclosed in the detailed description hereinafter.

Accordingly, at least part of the main objects of the present invention is achieved by a fragrance (perfume) and/or flavor oil containing particle, consisting of:
(a) 65 - 80 wt.% fragrance and/or flavor oil,
(b) 5 to 25 wt.% modified starch, wherein the modified starch is octenylsuccinated starch,
(c) 5 to 25 wt.% of one or more phosphate salts selected from mono alkaline metal phosphates,
(d) 0 - 10 wt.% of one or more additional ingredients,
wherein the weight percent values are based on the total dry weight of the particle and wherein the particles comprise no other modified starch.

Unless stated otherwise, in the present description of inventive particles, weight percent values of constituents of the inventive particles are based on the total dry (i.e. water-free) weight of the inventive particles.

A second subject matter of the present invention relates to a process for producing the inventive fragrance and/or flavor oil containing particles comprising or consisting of the following steps:
i. forming a mixture consisting of the following constituents (a) through (e)
   (a) 20 - 56 wt.% fragrance and/or flavor oil,
   (b) 2 to 17.5 wt.% modified starch, wherein the modified starch is octenylsuccinated starch
   (c) 2 to 17.5 wt.% of one or more phosphate salts selected from mono alkaline metal phosphates,
   (d) 0 - 7 wt.% of one or more additional ingredients and
   (e) 30 - 60 wt%., preferably 35 - 55 wt.%, of water,
   in each case based on the total weight of the mixture and then
(ii) drying the mixture of step i) to result in the fragrance and/or flavor oil containing particles, wherein the particles comprise no other modified starch.

A third subject matter of the present invention relates to a fragrance and/or flavor containing particle obtainable by the inventive process of production as set out hereinbefore.

A fourth subject matter of the present invention relates to a perfumed and/or flavored consumer product comprising one or more inventive fragrance and/or flavor oil containing particles.

A fifth subject matter of the present invention relates to a method of perfuming or flavoring a consumer product by adding one or more inventive fragrance and/or flavor oil containing particles to the consumer product.

A sixth subject matter of the present invention relates to a use of one or more of the inventive fragrance and/or flavor oil containing particles to perfume and/or flavor a consumer product.

All percentages, ratios and proportions herein are on a weight basis unless otherwise indicated.

The subject matter of the present invention as set out hereinbefore can also comprises in any possible combination all preferred embodiments as set out in the dependent claims or disclosed in the following detailed description.

### Detailed description of the present invention:

The present invention relates to a fragrance and/or flavor oil containing particle consisting of
   (a) 65 - 80 wt.%, preferably 65 - 78 wt.% fragrance and/or flavor oil,
   (b) 5 to 25 wt.% modified starch, preferably 10 - 25 wt.% chemically modified starch, wherein the modified starch is octenylsuccinated starch,
   (c) 5 to 25 wt.% of one or more phosphate salts selected from mono alkaline metal phosphates, preferably 5 - 15 wt.% of monosodium and/or monopotassium phosphate and
   (d) 0 - 10 wt.%, preferably 0 - 5 wt.% of one or more additional ingredients,
wherein the weight percent values are based on the total dry weight of the particle and wherein the particles comprise no other modified starch; as well as perfumed and/or flavored consumer products comprising the inventive particles.

It was surprisingly found by the inventors that the inventive blend of the specific phosphates salts selected from mono alkaline metal phosphates according constituent (c) and the modified starch ingredient according to constituent (b) wherein the modified starch is octenylsuccinated starch improves the emulsification capacity of the fragrance and/or flavor oil containing mixture and resulted in a fragrance and/or flavor oil loading of the octenylsuccinated starch far higher than the normal loading capacity.

Experiments encompassed different ratios of the constituents (a) through (c), namely the fragrance and/or flavor oil, the octenylsuccinated starch or the specific phosphates achieved (i) an fragrance and/or flavor oil loading up to 80 wt.% based on the total dry weight of the inventive particles, (ii) variable controlled release characteristics of the fragrance or flavor out of the inventive particles, (iii) free flowing inventive particles, (iv) a comparatively small particle size distribution of the inventive particles and (v) a comparatively very low surface oil content based on the total weight of the inventive particles. The high fragrance and/or flavor oil loading of up to 80 wt.-% allows for reducing the amount of the inventive particles when perfuming or flavoring consumer products and accordingly the costs, in particular the raw material costs can be lowered. The free flowability and the comparatively small particle size distribution of the inventive particles indicate that the particles can be handled easily when manufacturing consumer products. The comparatively low content of the surface oil indicates that the inventive particles are comparatively stable against oxidation as the content of surface oil directly correlates to the oxidation sensitivity. Accordingly, one or more of the main objects of the present invention are achieved by the inventive particles and, thus, the surprising characteristics of the inventive particles are all beneficial to the normal usage within consumer products containing microencapsulated fragrance or flavor oils.

The inventive fragrance and/or flavor oil containing particles are formed by drying techniques, preferably the spray-drying technique. Accordingly, the process of producing the inventive particles comprises or consists of the following steps:
i. forming a mixture consisting of the following constituents (a) through (e)
   (a) 20 - 56 wt.%, preferably 27.5 - 52 wt.%, fragrance and/or flavor oil,
   (b) 2 to 17.5 wt.%, preferably 2.25 - 11.25 wt.%, modified starch, wherein the modified starch is octenylsuccinated starch
   (c) 2 to 17.5 wt.%, preferably 2.25 - 11.25 wt.%, of one or more phosphate salts selected from mono alkaline metal phosphates,
   (d) 0 - 7 wt.%, preferably 0 - 6.5 wt.%, of one or more additional ingredients and
   (e) 30 - 60 wt%., preferably 35 - 55 wt.%, of water,
   in each case based on the total weight of the mixture and then
(ii) drying, preferably spray-drying, the mixture of step i) to result in the fragrance and/or flavor oil containing particles, and wherein the particles comprise no other modified starch.

The mixture mixture of step i) usually is an emulsion containing the octenylsuccinated starch, one or more of the phosphate salts, the fragrance and/or flavor oil and the water.

For producing the fragrance and/or flavor oil containing particles according to the present invention various known drying techniques or processes can be employed. The spray-drying technique preferably used herein to produce the preferred particles according to the present invention aerosolizes droplets of said mixture (usually an emulsion) in a heated environment which allows most of the water to evaporate, resulting in a virtually homogenous particle.

In the inventive spray-drying process, particles usually are produced by a three step operation comprising (1) forming an emulsion of the liquid core material in a solution, usually aqueous, of the normally solid coating material and (2) breaking up the emulsion into droplets of desired size, e.g., in a spray nozzle, from a spinning disc, or apertured centrifugal atomizer, and (3) removing moisture in a drying environment to solidify the coating material in the droplets to form solid particles. The drying environment preferably is hot drying air, e.g., in a spray-drying tower. The particles produced by this process which may be "hollow" or "solid", are characterized by cellular structure comprising many dispersed globules of the core material in a matrix of the coating material. "Solid" in this context means that a particle has more or less uniform structure throughout, as opposed to the "hollow" form of particle which has a shell surrounding a void, but it does not imply absence of pores or cells in the body thereof

One of the preferred processes for producing particles according to the present invention involves spraying the mixture of step i) of the inventive production process into a drying atmosphere to form globules or droplets. The products of this inventive process are dry, somewhat porous powders containing roughly spherical, convoluted particles with the coating material in the solid state and with the fragrance or flavor oil either dispersed as minute droplets throughout the particle, or dissolved in a solid matrix, or both, depending on the compatibility of the oil and coating material comprising modified starch wherein the modified starch is octenylsuccinated starch, and one or more mono alkaline metal phosphates.

In a further embodiment the inventive fragrance and/or flavor oil containing particles can be produced by continuous fluidized bed spray granulation, for example according to EP-A 163 836 or WO 00/36931.

The inventive fragrance and/or flavor oil containing particles in particular produced by continuous fluidized bed spray granulation are free flowing, have low dust generation, and are granular particles.

In accordance with the present invention the spray mixture of step i) of the inventive production process can be sprayed from below, from the side, or even from above into the fluidized bed.

Discontinuous fluidized bed spray granulation, for example according to EP-A 70 719, is feasible, but not preferred.

From the spray mixture of step i) of the inventive production process, the inventive particles are produced preferably with a particle size (particle diameter) of equal to or less than 300 micrometer (microns, µm). In a more preferred embodiment the inventive (preferably spray-dried) fragrance and/or flavor oil containing particles have an average particle size (median value) in the range of 5 to 125 microns, more preferably in the range of 5 to 80 microns, and most preferably in the range of 10 to 50 microns. The advantage of smaller differences in the size distribution of the inventive fragrance and/or flavor oil containing particles is that the inventive particles are less de-mixed in a further manufacturing step and have similar, comparatively uniform release properties regarding the fragrance and/or flavor oil.

In a preferred embodiment of the invention the particle size determination is conducted by laser diffraction (for example with the Beckman Coulter LS Particle Size Analyzer or the Master Sizer® MSS Longbench by Malvern Instruments Ltd.).

In a still further preferred embodiment the inventive (preferably spray-dried) fragrance and/or flavor oil containing particles have a residual water content of equal to or less than 3 wt.%, preferably the residual water content in the range of 0.1 to 2.5 wt.%, more preferably in the range of 0.3 to 2 wt.%, based on the total weight of the particle. The advantage correlated with a low water content relate to the fact that the inventive particles are less exposed to microorganism growth and, thus, the amount of preservatives in the inventive particles can be reduced.

In a still further preferred embodiment the inventive (preferably spray-dried) fragrance and/or flavor oil containing particles have an amount of surface oil of equal to or less than 4 wt.%, more preferably of less than 3 wt.%, most preferably of less than 2.5 wt.%, based on the total dry weight of the particle.

Accordingly, in a still further preferred embodiment the inventive (spray-dried) fragrance and/or flavor oil containing particles comprise or consist of:
(a) 65 - 78 wt.% fragrance and/or flavor oil,
(b) 10 - 25 wt.% chemically modified starch, wherein the modified starch is octenylsuccinated starch
(c) 5 - 15 wt.% of monosodium and/or monopotassium phosphate and
(d) 0 - 5 wt.% of one or more additional ingredients,
wherein the weight percent values are based on the total dry weight of the particle and wherein the particles comprise no other modified starch.

In a still more preferred embodiment the inventive (spray-dried) fragrance and/or flavor oil containing particles comprise or consist of:
(a) 65 - 75 wt.% fragrance or flavor oil,
(b) 15 - 22 wt.% of octenylsuccinated starch, wherein the modified starch is octenylsuccinated starch
(c) 8 - 12 wt.% of monosodium phosphate;
(d) 0 - 5 wt.% additional ingredients.
wherein the particles comprise no other modified starch.

In a still more preferred embodiment the inventive (spray-dried) fragrance and/or flavor oil containing have (in addition)
(i) an average particle size in the range of 5 to 125 microns, and/or
(ii) a residual water content in the range of 0.1 to 2.5 wt.%, and/or
(iii) an amount of surface oil of less than 3 wt.%,
wherein all weight percent values are based on the total dry weight of the particles.

The preferred embodiments of the inventive fragrance and/or flavor oil containing parti-cles and the inventive production process thereof can be combined in every possible way with each other and with the preferred embodiments of the invention as further set out hereinbelow.

In the following the preferred embodiments of constituents (a) through (d) of the inventive fragrance (perfume) and/or flavor oil containing particles are further disclosed:

### Constituent (a):

A fragrance (perfume) or flavor oil in the context of the present invention comprises at least one volatile fragrance and/or flavor substance (i.e. sensory active substance), but usually two or more different volatile fragrance and/or flavor substances. Fragrance or flavor oils typically are complex mixtures of volatile components. In particular, fragrance or flavor substances in the context of the present invention are sensorially active substances having a vapor pressure of at least 0,01 Pa at 25°C, more preferably a vapor pressure of at least 0,025 Pa at 25°C. Fragrance or flavor substances in large part show a vapor pressure of at least 0,5 Pa at 25°C, therefore such fragrance or flavor substances in particular are meant by the term volatile component.

The fragrance or flavor oil loading is in the range of 65 to 80 wt.%, preferably in the range of 65 to 78 wt.%, more preferably in the range of 65 to 75 wt.%, most preferably in the range of 65 to 75 wt.%, based on the total dry weight of the (preferably spray-dried) particle.

Preferably, the weight percent value of fragrance or flavor oil means the total weight percent value of volatile fragrance or flavor substances based on the total dry weight of the inventive particles.

Preferred volatile fragrance substances as part of the fragrance oil are:
Extracts of natural raw materials such as essential oils, concretes, absolutes, resins, resinoids, balsams, tinctures such as for example ambergris tincture; amyris oil; angelica seed oil; angelica root oil; aniseed oil; valerian oil; basil oil; wood moss absolute; bay oil; mugwort oil; benzoin resin; bergamot oil; beeswax absolute; birch tar oil; bitter almond oil; savory oil; bucco-leaf oil; cabreuva oil; cade oil; calamus oil; camphor oil; cananga oil; cardamom oil; cascarilla oil; cassia oil; cassia absolute; castoreum absolute; cedar-leaf oil; cedarwood oil; cistus oil; citronella oil; lemon oil; copaiba balsam; copaiba balsam oil; coriander oil; costus root oil; cumin oil; cypress oil; davana oil; dill oil; dillseed oil; eau de brouts absolute; oakmoss absolute; elemi oil; tarragon oil; eucalyptus citriodora oil; eucalyptus oil; fennel oil; fir oil; galbanum oil; galbanum resin; geranium oil; grapefruit oil; guaiac wood oil; gurjun balsam; gurjun balsam oil; helichrysum absolute; helichrysum oil; ginger oil; iris root abolute; iris root oil; jasmine absolute; calamus oil; blue camomile oil; Roman camomile oil; carrot-seed oil; cascarilla oil; pine-needle oil; spearmint oil; caraway oil; labdanum oil; labdanum absolute; labdanum resin; lavandin absolute; lavandin oil; lavender absolute; lavender oil; lemongrass oil; lovage oil; distilled lime oil; pressed lime oil; linaloe oil; litsea cubeba oil; bay-leaf oil; mace oil; marjoram oil; mandarin oil; massoi bark oil; mimosa absolute; ambrette oil; tincture of musk; clary sage oil; myristica oil; myrrh absolute; myrrh oil; myrtle oil; clove leaf oil; clove flower oil; neroli oil; olibanum abolute; olibanum oil; opopanax oil; orange blossom absolute; orange oil; origanum oil; palmarosa oil; patchouli oil; perilla oil; Peru balsam oil; parsley leaf oil; parsley seed oil; petitgrain oil; peppermint oil; pepper oil; pimento oil; pine oil; pennyroyal oil; rose absolute; rosewood oil; rose oil; rosemary oil; Dalmatian sage oil; Spanish sage oil; sandalwood oil; celery seed oil; spike lavender oil; Japanese aniseed oil; styrax oil; tagetes oil; fir-needle oil; tea-tree oil; turpentine oil; thyme oil; Tolu balsam; tonka absolute; tuberose absolute; vanilla extract; violet leaf absolute; verbena oil; vetiver oil; juniper oil; wine-lees oil; wormwood oil; wintergreen oil; ylang oil; hyssop oil; civet absolute; cinnamon leaf oil; cinnamon bark oil; as well as fractions thereof or constituents isolated therefrom;

Individual volatile fragrance substances are preferably selected from the group consisting of:
- Hydrocarbons, preferably 3-carene; α-pinene; β-pinene; α-terpinene; γ-terpinene; p-cymene; bisabolene; camphene; caryophyllene, cedrene; farnesene; liminene; longifolene; myrcene; ocimene; valencene; (E,Z)-1,3,5-undecatriene;
- Aliphatic alcohols, preferably hexanol; octanol; 3-octanol; 2,6-dimethylheptanol; 2-methylheptanol; 2-methyloctanol; (E)-3-hexenol; (E) and (Z)-3-hexenol; 1-octen-3-ol; mixtures of 3,4,5,6,6-pentamethyl-3/4-hepten-2-ol and 3,5,6,6-tetramethyl-4-methyleneheptan-2-ol; (E,Z)-2,6-nonadienol; 3,7-dimethyl-7-methoxyoctan-2-ol; 9-decenol; 10-undecenol; 4-methyl-3-decen-5-ol;
- Alphatic aldehydes and their acetals, preferably hexanal; heptanal; octanal; nonanal; decanal; undecanal; dodecanal; tridecanal; 2-methyloctanal; 2-methylnonanal; (E)-2-hexenal; (Z)-4-heptenal; 2,6-dimethyl-5-heptenal; 10-undecenal; (E)-4-decenal; 2-dodecenal; 2,6,10-trimethyl-5,9-undecadienal; heptanal diethyl acetal; 1,1-dimethoxy-2,2,5-trimethyl-4-hexene; citronellyl oxyacetaldehyde;
- Aliphatic ketones and oximes thereof, preferably 2-heptanone; 2-octanone; 3-octanone; 2-nonanone; 5-methyl-3-heptanone; 5-methyl-3-heptanone oxime; 2,4,4,7-tetramethyl-6-octen-3-one;
- Aliphatic sulphur-containing compounds, preferably 3-methylthiohexanol; 3-methylthiohexyl acetate; 3-mercaptohexanol; 3-mercaptohexyl acetate; 3-mercaptohexyl butyrate; 3-acetylthiohexyl acetate; 1-menthene-8-thiol;
- Aliphatic nitriles, preferably 2-nonenenitrile; 2-tridecenenenitrile; 2,12-tridecenene-nitrile; 3,7-dimethyl-2,6-octadienenitrile; 3,7-dimethyl-6-octenenitrile;
- Aliphatic carboxylic acids and esters thereof, preferably (E)- and (Z)-3-hexenyl formate; ethyl acetoacetate; isoamyl acetate; hexyl acetate; 3,5,5-trimethylhexyl acetate; 3-methyl-2-butenyl acetate; (E)-2-hexenyl acetate; (E)- and (Z)-3-hexenyl acetate; octyl acetate; 3-octyl acetate; 1-octen-3-yl acetate; ethyl butyrate; butyl butyrate; isoamyl butyrate; hexyl butyrate; (E)- and (Z)-3-hexenyl isobutyrate; hexyl crotonate; ethyl isovalerate; ethyl 2-methylpentanoate; ethyl hexanoate; allyl hexanoate; ethyl heptanoate; allyl heptanoate; ethyl octanoate; ethyl (E,Z)-2,4-decadienoate; methyl 2-octynoate; methyl 2-nonynoate; allyl-2-isoamyloxyacetate; methyl-3,7-dimethyl-2,6-octadienoate;
- Acyclic terpene alcohols, preferably citronellol; geraniol; nerol; linalool; lavandulol; nerolidol; farnesol; tetrahydrolinalool; tetrahydrogeraniol; 2,6-dimethyl-7-octen-2-ol; 2,6-dimethyloctan-2-ol; 2-methyl-6-methylene-7-octen-2-ol; 2,6-dimethyl-5,7-octadien-2-ol; 2,6-dimethyl-3,5-octadien-2-ol; 3,7-dimethyl-4,6-octadien-3-ol; 3,7-dimethyl-1,5,7-octatrien-3-ol; 2,6-dimethyl-2,5,7-octatrien-1-ol; and formates, acetates, propionates, isobutyrates, butyrates, isovalerates, pentanoates, hexanoates, crotonates, tiglinates, 3-methyl-2-butenoates thereof;
- Acyclic terpene aldehydes and ketones, preferably geranial; neral; citronellal; 7-hydroxy-3,7-dimethyloctanal; 7-methoxy-3,7-dimethyloctanal; 2,6,10-trimethyl-9-undecenal; geranylacetone; and the dimethyl and diethyl acetals of geranial, neral, 7-hydroxy-3,7-dimethyloctanal;
- Cyclic terpene alcohols, preferably menthol; isopulegol; alpha-terpineol; terpineol-4; menthan-8-ol; menthan-1-ol; menthan-7-ol; borneol; isoborneol; linalool oxide; nopol; cedrol; ambrinol; vetiverol; guaiol; and the formates, acetates, propionates, isobutyrates, butyrates, isovalerates, pentanoates, hexanoates, crotonates, tiglinates, 3-methyl-2-butenoates thereof;
- Cyclic terpene aldehydes and ketones, preferably menthone; isomenthone; 8-mercaptomenthan-3-one; carvone; camphor; fenchone; alpha-ionone; beta-ionone; alpha-n-methylionone; beta-n-methylionone; alpha-isomethylionone; beta-isomethylionone; alpha-irone; alpha-damascone; beta-damascone; beta-damascenone; delta-damascone; gamma-damascone; 1-(2,4,4-trimethyl-2-cyclohexen-1-yl)-2-buten-1-one; 1,3,4,6,7,8a-hexahydro-1,1,5,5-tetramethyl-2H-2,4a-methanonaphthalen-8(5H)-one; nootkatone; dihydronootkatone; alpha-sinensal; beta-sinensal; acetylated cedarwood oil (methyl cedryl ketone);
- Cyclic alcohols, preferably 4-tert.-butylcyclohexanol; 3,3,5-trimethylcyclohexanol; 3-isocamphylcyclohexanol; 2,6,9-trimethyl-Z2,Z5,E9-cyclododecatrien-1-ol; 2-isobutyl-4-methyltetrahydro-2H-pyran-4-ol;
- Cycloaliphatic alcohols, preferably alpha-3,3-trimethylcyclohexylmethanol; 2-methyl-4-(2,2,3-trimethyl-3-cyclopent-1-yl)butanol; 2-methyl-4-(2,2 ,3-trimethyl-3-cyclopent-1-yl)-2-buten-1-ol; 2-ethyl-4-(2,2,3-trimethyl-3-cyclopent-1-yl)-2-buten-1-ol; 3-methyl-5-(2,2,3-trimethyl-3-cyclopent-1-yl)-pentan-2-ol; 3-methyl-5-(2,2,3-timethyl-3-cyclopent-1-yl)-4-penten-2-ol; 3,3-dimethyl-5-(2,2,3-trimethyl-3-cyclopent-1-yl)-4-penten-2-ol; 1-(2,2,6-trimethylcyclohexyl)pentan-3-ol; 1-(2,2,6-trimethylcyclohexyl)hexan-3-ol;
- Cyclic and cycloaliphatic ethers, preferably cineol; cedryl methyl ether; cyclododecyl methyl ether; (ethoxymethoxy)cyclododecane; alpha-cedrene epoxide; 3a,6,6,9a-tetramethyl-dodecahydronaphtho[2,1-b]furan; 3a-ethyl-6,6,9a-trimethyldodecahydro-naphtho[2,1-b]furan; 1,5,9-trimethyl-13-oxabicyclo[10.1.0]trideca-4,8-diene; rose oxide; 2-(2,4-dimethyl-3-cyclohexen-1-yl)-5-methyl-5-(1-methylpropyl)-1,3-dioxane;
- Cyclic ketones, preferably 4-tert.-butylcyclohexanone; 2,2,5-trimethyl-5-pentylcyclopentanone; 2-heptylcyclopentanone; 2-pentylcyclopentanone; 2-hydroxy-3-methyl-2-cyclopenten-1-one; 3-methyl-cis-2-penten-1-yl-2-cyclopenten-1-one; 3-methyl-2-pentyl-2-cyclopenten-1-one; 3-methyl-4-cyclopentadecenone; 3-methyl-5-cyclopentadecenone; 3-methylcyclopentadecanone; 4-(1-ethoxyvinyl)-3,3,5,5-tetramethylcyclohexanone; 4-tert.-pentylcyclohexanone; 5-cyclohexadecen-1-one; 6,7-dihydro-1,1,2,3,3-pentamethyl-4(5H)-indanone; 9-cycloheptadecen-1-one; cyclopentadecanone; cyclohexadecanone;
- Cycloaliphatic aldehydes, preferably 2,4-dimethyl-3-cyclohexenecarbaldehyde; 2-methyl-4-(2,2,6-trimethyl-cyclohexen-1-yl)-2-butenal; 4-(4-hydroxy-4-methylpentyl)-3-cyclohexenecarbaldehyde; 4-(4-methyl-3-penten-1-yl)-3-cyclohexenecarbaldehyde;
- Cycloaliphatic ketones, preferably 1-(3,3-dimethylcyclohexyl)-4-penten-1-one; 1-(5,5-dimethyl-1-cyclohexen-1-yl)-4-penten-1-one; 2,3,8,8-tetramethyl-1,2,3,4,5,6,7,8-octahydro-2-naphthalenyl methyl ketone; methyl-2,6,10-trimethyl-2,5,9-cyclododecatrienyl ketone; tert.-butyl (2,4-dimethyl-3-cyclohexen-1-yl) ketone;
- Esters of cyclic alcohols, preferably 2-tert.-butylcyclohexyl acetate; 4-tert.-butylcyclohexyl acetate; 2-tert.-pentylcyclohexyl acetate; 4-tert.-pentylcyclohexyl acetate; decahydro-2-naphthyl acetate; 3-pentyltetrahydro-2H-pyran-4-yl acetate; decahydro-2,5,5,8a-tetramethyl-2-naphthyl acetate; 4,7-methano-3a,4,5,6,7,7a-hexahydro-5 or 6-indenyl acetate; 4,7-methano-3a,4,5,6,7,7a-hexahydro-5 or 6-indenyl propionate; 4,7-methano-3a,4,5,6,7,7a-hexahydro-5 or 6-indenyl isobutyrate; 4,7-methanooctahydro-5 or 6-indenyl acetate;
- Esters of cycloaliphatic carboxylic acids, preferably allyl 3-cyclohexylpropionate; allyl cyclohexyloxyacetate; methyl dihydrojasmonate; methyl jasmonate; methyl 2-hexyl-3-oxocyclopentanecarboxylate; ethyl 2-ethyl-6,6-dimethyl-2-cyclohexenecarboxylate; ethyl 2,3,6,6-tetramethyl-2-cyclohexenecarboxylate; ethyl 2-methyl-1,3-dioxolane-2-acetate;
- Aromatic hydrocarbons, preferably styrene and diphenylmethane;
- Araliphatic alcohols, preferably benzyl alcohol; 1-phenylethyl alcohol; 2-phenylethyl alcohol; 3-phenylpropanol; 2-phenylpropanol; 2-phenoxyethanol; 2,2-dimethyl-3-phenylpropanol; 2,2-dimethyl-3-(3-methylphenyl)propanol; 1,1-dimethyl-2-phenylethyl alcohol; 1,1-dimethyl-3-phenylpropanol; 1-ethyl-1-methyl-3-phenylpropanol; 2-methyl-5-phenylpentanol; 3-methyl-5-phenylpentanol; 3-phenyl-2-propen-1-ol; 4-methoxybenzyl alcohol; 1-(4-isopropylphenyl)ethanol;
- Esters of araliphatic alcohols and aliphatic carboxylic acids, preferably benzyl acetate; benzyl propionate; benzyl isobutyrate; benzyl isovalerate; 2-phenylethyl acetate; 2-phenylethyl propionate; 2-phenylethyl isobutyrate; 2-phenylethyl isovalerate; 1-phenylethyl acetate; alpha-trichloromethylbenzyl acetate; alpha, alpha-dimethylphenylethyl acetate; alpha,alpha-dimethylphenylethyl butyrate; cinnamyl acetate; 2-phenoxyethyl isobutyrate; 4-methoxybenzyl acetate; araliphatic ethers such as for example 2-phenylethyl methyl ether; 2-phenylethyl isoamyl ether; 2-phenylethyl 1-ethoxyethyl ether; phenylacetaldehyde dimethyl acetal; phenylacetaldehyde diethyl acetal; hydratropaldehyde dimethyl acetal; phenylacetaldehyde glycerol acetal; 2,4,6-trimethyl-4-phenyl-1,3-dioxane; 4,4a,5,9b-tetrahydroindeno[1,2-d]-m-dioxin; 4,4a,5,9b-tetrahydro-2,4-dimethylindeno[1,2-d]-m-dioxin;
- Aromatic and araliphatic aldehydes, preferably benzaldehyde; phenylacetaldehyde; 3-phenylpropanal; hydratropaldehyde; 4-methylbenzaldehyde; 4-methylphenylacetaldehyde; 3-(4-ethylphenyl)-2,2-dimethylpropanal; 2-methyl-3-(4-isopropylphenyl)propanal; 2-methyl-3-(4-tert.-butylphenyl)propanal; 3-(4-tert.-butylphenyl)propanal; cinnamaldehyde; alpha-butylcinnamaldehyde; alpha-amylcinnamaldehyde; alpha-hexylcinnamaldehyde; 3-methyl-5-phenylpentanal; 4-methoxybenzaldehyde; 4-hydroxy-3-methoxybenzaldehyde; 4-hydroxy-3-3,4-methylenedioxybenzaldehyde; 3,4-dimethoxy-benzaldehyde; 2-methyl-3-(4-methoxyphenyl)propanal; 2-methyl-3-(4-methylenedioxyphenyl)propanal;
- Aromatic and araliphatic ketones, preferably acetophenone; 4-methylacetophenone; 4-methoxyacetophenone; 4-tert.-butyl-2,6-dimethylacetophenone; 4-phenyl-2-butanone; 4-(4-hydroxyphenyl)-2-butanone; 1-(2-naphthalenyl)ethanone; benzophenone; 1,1,2,3,3,6-hexamethyl-5-indanyl methyl ketone; 6-tert.-butyl-1,1-dimethyl-4-indanyl methyl ketone; 1-[2,3-dihydro-1,1,2,6-tetramethyl-3-(1-methylethyl)-1H-5-indenyl]ethanone; 5',6',7',8'-tetrahydro-3',5',5',6',8',8'-hexamethyl-2-acetonaphthone;
- Aromatic and araliphatic carboxylic acids and esters thereof, preferably acid; phenylacetic acid; methyl benzoate; ethyl benzoate; hexyl benzoate; benzyl benzoate; methyl phenylacetate; ethyl phenylacetate; geranyl phenylacetate; phenylethyl phenylacetate; methyl cinnamate; ethyl cinnamate; benzyl cinnamate; phenylethyl cinnamate; cinnamyl cinnamate; allyl phenoxyacetate; methyl salicylate; isoamyl salicylate; hexyl salicylate; cyclohexyl salicylate; cis-3-hexenyl salicylate; benzyl salicylate; phenylethyl salicylate; methyl 2,4-dihydroxy-3,6-dimethylbenzoate; ethyl 3-phenylglycidate; ethyl 3-methyl-3-phenylglycidate;
- Nitrogen-containing aromatic compounds, preferably 2,4,6-trinitro-1,3-dimethyl-5-tert.-butylbenzene; 3,5-dinitro-2,6-dimethyl-4-tert.-butylacetophenone; cinnamonitrile; 5-phenyl-3-methyl-2-pentenenitrile; 5-phenyl-3-methylpentanenitrile; methyl anthranilate; methyl N-methylanthranilate; Schiff bases of methyl anthranilate with 7-hydroxy-3,7-dimethyloctanal; 2-methyl-3-(4-tert.-butylphenyl)propanal or 2,4-dimethyl-3-cyclohexenecarbaldehyde; 6-isopropylquinoline; 6-isobutylquinoline; 6-sec.-butylquinoline; indole; skatole; 2-methoxy-3-isopropylpyrazine; 2-isobutyl-3-methoxypyrazine;
- Phenols, phenyl ethers and phenyl esters, preferably estragole; anethole; eugenol; eugenyl methyl ether; isoeugenol; isoeugenyl methyl ether; thymol; carvacrol; diphenyl ether; beta-naphthyl methyl ether; beta-naphthyl ethyl ether; beta-naphthyl isobutyl ether; 1,4-dimethoxybenzene; eugenyl acetate; 2-methoxy-4-methylphenol; 2-ethoxy-5-(1-propenyl)phenol; p-cresyl phenylacetate;
- Heterocyclic compounds, preferably 2,5-dimethyl-4-hydroxy-2H-furan-3-one; 2-ethyl-4-hydroxy-5-methyl-2H-furan-3-one; 3-hydroxy-2-methyl-4H-pyran-4-one; 2-ethyl-3-hydroxy-4H-pyran-4-one;
- Lactones, preferably 1,4-octanolide; 3-methyl-1,4-octanolide; 1,4-nonanolide; 1,4-decanolide; 8-decen-1,4-olide; 1,4-undecanolide; 1,4-dodecanolide; 1,5-decanolide; 1,5-dodecanolide; 1,15-pentadecanolide; cis and trans-11-pentadecen-1,15-olide; cis- and trans-12-pentadecen-1,15-olide; 1,16-hexadecanolide; 9-hexadecen-1,16-olide; 10-oxa-1,16-hexadecanolide; 11-oxa-1,16-hexadecanolide; 12-oxa-1,16-hexadecanolide; ethylene 1,12-dodecanedioate; ethylene 1,13-tridecanedioate; coumarin; 2,3-dihydrocoumarin; octahydrocoumarin.

Fragrance oils used in the context of the present invention may contain one or more of the following solvents / diluents: ethanol, isopropanol, diethylene glycol monoethyl ether, glycerol, propylene glycol, 1,2-butylene glycol, dipropylene glycol, diethyl phthalate, triethyl citrate, isopropyl myristate and the like. These solvents / diluents are then considered as being comprised in constituent (a).

Flavor oils used in the context of the present invention may contain the following solvents / diluents: ethanol, vegetable oil triglycerides, 1,2-propylene glycol, benzyl alcohol, tri-acetin (glycerol triacetate), diacetin (glycerol diacetate), triethyl citrate, glycerol. These solvents / diluents are then considered as being comprised in constituent (a).

Preferred flavor oils are, for example, essential oils, fractions thereof, or individual aroma substances. Examples which may be mentioned are: extracts from natural raw materials, such as essential oils, concretes, absolutes, resins, resinoids, balsams and tinctures, such as aniseed oil; basil oil; bergamot oil; bitter almond oil; camphor oil; lemon oil; eucalyptus oil; geranium oil; grapefruit oil; ginger oil; camomile oil; spearmint oil, caraway oil, lime oil; mandarin oil; clove (blossom) oil, orange oil; peppermint oil; rose oil; rosemary oil; sage oil; yarrow oil; star aniseed oil; thyme oil; vanilla extract; juniper berry oil; wintergreen oil; cinnamon leaf oil; cinnamon bark oil; and fractions thereof and constituents isolated therefrom.

Individual volatile flavor substances, particularly useful in oral care compositions, are preferably selected from the group consisting of anethol, menthol, I-menthol, menthone, isomenthone, menthyl acetate, menthofuran, menthyl methylether, mintlactone, eucalyptol, limonene, eugenol, alpha-pinene, beta-pinene, cis-sabinene hydrate, 3-octanol, I-carvone, gamma-octalactone, gamma-nonalactone, germacrene-D, viridiflorol, 1,3E,5Z-undecatriene, isopulegol, piperitone, 2-butanone, ethyl formiate, 3-octyl acetate, isoamyl isovalerianate, hexanol, hexanal, cis-3-hexenol, linalool, alpha-terpineol, cis- / transcarvyl acetate, p-cymol, thymol, 4,8-dimethyl-3,7-nonadien-2-one, damascenone, damascone, rose oxide, dimethyl sulfide, fenchol, acetaldehyde diethylacetal, cis-4-heptenal, isobutyraldehyde, isovaleraldehyde, cis-jasmone, anisaldehyde, methyl salicylate, myrtenyl acetate, 8-ocimenyl acetate, 2-phenylethyl alcohol, 2-phenylethyl isobutyrate, 2-phenylethyl isovalerate, cinnamic aldehyde, geraniol and nerol.

The preferred embodiments of the fragrance and flavor oil of constituent (a) can be combined in any possible way with each other and/or with any of the (preferred) embodiments of the constituents (b) through (d) of the inventive fragrance and/or flavor oil containing particles as well as with the further subject matter of the present invention including the respective preferred embodiments thereof.

### Constituent (b):

The modified starch of constituent (b) is a chemically modified starch wherein the modified starch is octenylsuccinated starch. Preferred modified starches are based on maize or corn (e.g. waxy or dent maize).

Octenylsuccinated starch (CAS-number 125109-81-1, chemical name: amylopectin, hydrogen 1-octadecenylbutanedioate; E1450), is e.g. commercially available as Hi-Cap 100 from National Starch). With octenylsuccinated starch the highest levels of loading of fragrance or flavor oil in the particles according to the present invention is achievable, said particles at the same time having a very low amount of surface oil and/or a very low residual water content.

The total amount of (chemically) modified starch as constituent (b) starch wherein the modified starch is octenylsuccinated starch is in the range of 5 to 25 wt.%, preferably in the range of 10 to 25 wt.%, more preferably in the range of 15 to 22 wt.%, based on the total dry weight of the (preferably spray-dried) particle.

The preferred embodiments of constituent (b) can be combined in any possible way with each other and/or with any of the (preferred) embodiments of the constituents (a), (c) and (d) of the inventive fragrance and/or flavor oil containing particles as well as with the further subject matter of the present invention including the respective preferred embodiments thereof.

### Constituent (c):

Constituent (c) is the mono alkaline metal phosphate salt corresponding to the formula MH₂PO₄, M being an alkaline metal. Preferably constituent (c) is a phosphate salt selected from monosodium phosphate NaH₂PO₄ and/or monopotassium phosphate KH₂PO₄. Preferred is monosodium phosphate.

The total amount of phosphate salts of constituent (c) is in the range of 5 to 25 wt.%, preferably in the range of 5 to 15 wt.%, more preferably in the range of 8 to 12 wt.%, based on the total dry weight of the (preferably spray-dried) particle.

When the (spray-) dried particles contain noticeably less than 5 wt.% of phosphate salts (in particular less than 4 wt.%), these particles were found not to be free-flowing.

The weight ratio of constituent (b) : constituent (c) preferably lies in the range of 4 : 1 - 1 : 3, more preferably in the range of 3 : 2 - 1 : 2, most preferably in the range of 2 : 1 - 1 : 1.

The preferred embodiments of constituent (c) can be combined in any possible way with each other and/or with any of the (preferred) embodiments of the constituents (a), (b) and (d) of the inventive fragrance and/or flavor oil containing particles as well as with the further subject matter of the present invention including the respective preferred embodiments thereof.

### Optional Additional Constituents (d):

In a preferred embodiment the total amount of optional additional constituents (d) is preferably in the range of 0 - 5 wt.%.

In a further preferred embodiment the optional ingredients and additives according to constituent (d) of the inventive fragrance and/or flavor oil containing particles are, e.g., selected from the following group:
preservatives, abrasives, anti-acne agents, anti-skin ageing agents, antibacterial agents, anticellulite agents, anti-dandruff agents, anti-inflammatory agents, irritation-preventing or inhibiting agents, antimicrobial agents, antioxidants, astringents, antiperspirants, antiseptics, antistatics, binders, buffers, carriers, chelating agents, cell stimulants, cleaning agents, caring agents, depilatories, surface-active agents, deodorants, antiperspirants, softeners, emulsifiers, enzymes, essential oils, fibres, film-formers, fixatives, flow-agents (anti-caking agents), foaming agents, foam stabilisers, foam-suppressors, foam boosters, fungicides, gelling agents, gel-forming agents, hair-care agents, hair-shaping agents, hair-straightening agents, moisturising agents, wetting agents, humectants, bleaching agents, starching agents, stain-removing agents, optical brighteners, water-proofing agents, soil-repelling agents, friction-reducing agents, lubricants, moisture creams, ointments, clouding agents, plasticizers, opacifiers, polishes, glossing agents, polymers, powders, proteins, refatting agents, exfoliating agents, silicones, skin-soothing agents, skin-cleansing agents, skin-care agents, skin-healing agents, skin-lightening agents, skin-protecting agents, skin softening agents, cooling agents, skin-cooling agents, heating agents, skin-heating agents, stabilisers, UV-absorbing agents, UV-filters, laundry detergents, fabric softeners, suspending agents, skin-tanning agents, thickeners, vitamins, minerals, oils, waxes, fats, phospholipids, saturated fatty acids, monounsaturated or polyunsaturated fatty acids, α-hydroxy acids, polyhydroxy fatty acids, dyes, coloring agents, color-protection agents, pigments, anti-corrosives, sweeteners, food acids, polyols, surfactants, electrolytes, organic solvents or silicone derivatives.

In a still further preferred embodiment one or more of the following preservatives are comprised in constituent (d) in the inventive fragrance and/or flavor oil particles: sodium chloride, sucrose, nitrites, preferably Na, K and/or Ca nitrites and sulfites, more preferably Na, K and/or Ca sulfites; organic acids or salts thereof, preferably sorbic acid, benzoic acid, formic acid and the Na, K and Ca salts of these acids, as well as 4-hydroxybenzoic acid esters, salicylic acid and dehydracetic acid. The total amount of the one or more preservatives as set out hereinbefore is preferably effective to preserve the inventive fragrance and/or flavor oil particles.

Still further preferred are one or more preservative acids selected from the group consisting of: citric acid, adipic acid, malic acid, fumaric acid, ascorbic acid (vitamin C), succinic acid and tartaric acid, more preferred are citric acid, ascorbic acid and malic acid.

In a further preferred embodiment one or more cooling agents are comprised in constituent (d) of the particles according to the present invention and are preferably selected from the group consisting of: menthone glycerine acetal, menthyl lactate, more preferably I-menthyl I-lactate, substituted menthyl-3-carboxylic acid amides (e.g. menthyl-3-carboxylic acid-N-ethylamide, WS-3), 2-isopropyl-N-2,3-trimethyl butanamide (WS-23), substituted cyclohexane carboxylic acid amides, 3-menthoxypropane-1,2-diol, 2-hydroxyethyl menthyl carbonate, 2-hydroxypropyl menthyl carbonate, N-[[5-methyl-2-(1-methylethyl)cyclohexyl]-carbonyl]glycine ethyl ester (WS-5), isopulegol, menthyl hydroxycarboxylic acid esters (e.g. menthyl-3-hydroxybutyrate), monomenthyl succinate, 2-mercaptocyclodecanone, menthyl-2-pyrrolidin-5-one carboxylate, 2,3-dihydroxy-p-menthane, 3,3,5-trimethyl cyclohexanone glycerine ketal, 3-menthyl-3,6-di- and trioxaalkanoates, 3-menthyl methoxyacetate, and icilin.

In a still further preferred embodiment one or more antioxidants, or substances which can intensify an antioxidative action, are suitable to be comprised in constituent (d) of the particles according to the invention and are preferably selected from the group consisting of: naturally occurring tocopherols and derivatives thereof, tocotrienols, flavonoids, ascorbic acid and its salts, alpha-hydroxy acids (e.g. citric acid, lactic acid, malic acid, tartaric acid) and Na, K and Ca salts thereof, constituents, extracts and fractions thereof isolated from plants, e.g. from tea, green tea, algae, grape seeds, wheat germ, rosemary and oregano; flavonoids, quercetin and phenolic benzylamines. Propyl gallate, octyl gallate, dodecyl gallate, butylhydroxyanisole (BHA), butylhydroxytoluene (BHT), lecithins, mono- and diglycerides of edible fatty acids esterified with citric acid, orthophosphates and Na, K and Ca salts of monophosphoric acid and ascorbyl palmitate are furthermore suitable as antioxidants.

In a still further preferred embodiment one or more flow-agents (anti-caking agent), such as silica (SiO₂), are comprised in constituent (d) to additionally improve the already good flowing properties and/or to additionally avoid caking or agglomeration of the particles of the present invention.

In a still further preferred embodiment one or more sugar alcohols are comprised in constituent (d) of the inventive particles and are preferably selected from the group consisting of: erythritol, threitol, arabitol, ribitol, xylitol, sorbitol, mannitol, dulcitol and lactitol. In a more preferred embodiment mannitol is comprised in constituent (d).

In a still further preferred embodiment one or more sweeteners are comprised in constituent (d) of the inventive particles and are preferably selected from the group consisting of: saccharin (optionally as the Na, K or Ca salt), aspartame (e.g. NutraSweet^{®}), cyclamate (optionally as the Na or Ca salt), acesulfame K (e.g. Sunett^{®}), thaumatin and/or neohesperidin Dihydrochalcone. It is of course also possible to use alternatively or in addition one or more other preferred sweeteners selected from the group consisting of: stevioside, rebaudioside A, glycyrrhizin, ultrasweet, osladin, brazzein, miraculin, pentadin, phyllodulcin, dihydrochalcones, arylureas, trisubstituted guanidines, glycyrrhizin, superaspartame, sucralose (trichlorogalactosucrose, TGS), alitame, monellin or Neotame^{®}.

In a still further preferred embodiment one or more substances of the group of dyestuffs, coloring agents and/or pigments are comprised in constituent (d). In the group of coloring agents the water-soluble coloring agents are preferred and most preferably one or more water-soluble coloring agents are selected from the group consisting of:

Tartrazine (E 102), Sunset Yellow (E 110), Carmoisine (E 122), Ponceau 4R (E 124), Amaranth (E 123), Allura Red (E 129), Brilliant Blue FCF (E 133), Indigo Carmine (E 132), Erythrosine (E 127), Black PN (E 151), Chocolate Brown HT (E155), Patent Blue V (E 131), Quinoline Yellow (E 104) and Green S (E 142);

FD&C Water Soluble Dyes: FD&C Blue No. 2, FD&C Blue No. 1, FD&C Green No. 3, FD&C Red No. 3,. FD&C Red No. 40, FD&C Yellow No. 6, FD&C Yellow No. 5, FD&C Red No. 4;

D&C Water Soluble: D&C Green 5, D&C Green 6, D&C Green 8, D&C Orange 4, D&C Red 17, D&C Red 28, D&C Red 33, D&C Red 34, D&C Red 36, D&C Red 6, D&C Red 7, D&C Red 8, D&C Yellow 10, D&C Yellow 11, D&C Yellow 8.

The preferred embodiments of constituent (d) can be combined in any possible way with each other and/or with any of the (preferred) embodiments of the constituents (a), (b) and (c) of the inventive fragrance and/or flavor oil containing particles as well as with the further subject matter of the present invention including the respective preferred embodiments thereof.

In accordance with the fourth subject matter of the present invention the inventive fragrance and/or flavor oil containing particles are comprised in perfumed and/or flavored consumer products. In the following the preferred embodiments of consumer products are disclosed:

In a preferred embodiment the inventive perfumed consumer products comprise one or more inventive fragrance oil containing particles and are preferably selected from the group consisting of: shaving lotions, pre-shave products, splash colognes and perfumed wipes, as well as for fragrancing acidic, alkaline or neutral cleaning products such as for example floor cleaners, window cleaners, dishwashing detergents, bath cleaners and sanitizers, abrasive creams, solid and liquid toilet cleaners, powdered and mousse carpet cleaners, liquid detergents, powder detergents, laundry pre-treatment products such as bleaches, soaking products and stain removers, fabric conditioners, laundry soaps, laundry tablets, disinfectants, hard surface disinfectants as well as air improvers in liquid or gel form or applied to a solid carrier, aerosol sprays, waxes and polishes such as furniture polishes, floor waxes, shoe creams as well as toiletries such as for example solid and liquid soaps, shower gels, shampoos, shaving soaps, shaving foams, bath oils, cosmetic emulsions of the oil-in-water, water-in-oil, and water-in-oil-in-water type, such as for example skin creams and lotions, face creams and lotions, sun-protection creams and lotions, after-sum creams and lotions, hand creams and lotions, foot creams and lotions, depilatory creams and lotions, after-shave creams and lotions, tanning creams and lotions, hair-care products such as for example hairsprays, hair gels, hair lotions, hair conditioners, permanent and semi-permanent hair dyes, hair-shaping products such as cold waving and hair-straightening products, hair tonics, hair creams and lotions, deodorants and antiperspirants such as for example underarm sprays, roll-ons, stick deodorants, cream deodorants, or decorative cosmetic products, candles and insect repellents.

Still further preferred inventive perfumed products are selected from the group consisting of:
- cleaning products: dishwashing detergents, bath and sanitary cleaners, scouring milk, solid WC cleaners, powder and foam carpet cleaners, powdered laundry detergents, fabric softeners (see US 5,500,138) fabric softener sheets, laundry pretreatment agents, bleaches, soaking agents, stain removers, washing soaps, washing tablets, disinfectants, surface disinfectants;
- air freshening products: air fresheners in gel form, air fresheners in solid carrier, aerosol sprays;
- waxes and polishes: furniture polishes, floor waxes, shoe creams;
- bodycare compositions: solid soaps, shower gels, shampoos, shaving foams, cosmetic emulsions of the oil-in-water, water-in-oil and water-in-oil-in-water type, such as, for example, skin creams and lotions, face creams and lotions, sunscreen creams and lotions, aftersun creams and lotions, hand creams and lotions, foot creams and lotions, depilatory creams and lotions, tanning creams and lotions;
- haircare products: hairsprays, hair gels, hairsetting lotions, permanent and semipermanent hair colorants, hair-shaping compositions;
- deodorants and antiperspirants: underarm sprays, roll-ons, deodorant sticks, deodorant creams; and
- decorative cosmetics: powdered make-up, foundations, lipsticks, mascara.

Preferred inventive perfumed products are low-moisture or dry products, i.e. products having a total water content of less than 25 %, more preferred of less than 15 %, even more preferred of less than 10%, based on the total weight of the product.

If the free water content of a product is comparatively high, the particles of the present invention can dissolve in the product during storage of the product and decrease in performance. Therefore, in a preferred embodiment, the inventive perfumed products have a free water content of less than 5 %, based on the total weight of the product.

Still more preferred inventive perfumed products are selected from the group consisting of: underarm deodorants and antiperspirants, solid sticks and aerosols, powdered laundry detergents, powdered carpet deodorizers, cat litter, adult incontinence diapers, baby diapers, bath salts and body talc.

Most preferred inventive perfumed products are selected from the group consisting of: underarm deodorants and antiperspirants, solid sticks and aerosols, fabric softener sheets and powdered laundry detergents.

Regarding typical ingredients, actives and their respective amounts in preferred compositions see US 6,123,932 for underarm deodorants, US 5,348,667 for fabric softener sheets and US 6,491,728 for powdered laundry detergents.

### Detergent Compositions

The preferred detergent according to the present invention is a powder or bar in solid form without water. A detergent composition according to the present invention preferably comprises the following ingredients: free perfume oil, particles of the present invention, one or more detersive surfactant, one or more detergency builder and optionally one or more additives as mentioned below.

### A. Detersive Surfactants

The detergent composition comprises from about 0.01% to about 95%, preferably from about 5% to about 85%, more preferably from about 3% to about 30%, and even more preferably from about 5% to about 22%, of a surfactant system. Detersive surfactants utilized can be of the anionic, nonionic, zwitterionic, ampholytic or cationic type or can comprise compatible mixtures of these types. Detergent surfactants useful herein are described in US 3,664,961, US 3,919,678, US 4,222,905, and in US 4,239,659.

Of the surfactants, anionics and nonionics are preferred and anionics are most preferred. Such preferred anionic surfactants can themselves be of several different types. For example, water-soluble salts of the higher fatty acids, i.e., "soaps", are useful anionic surfactants in the compositions herein. This includes alkali metal soaps such as the sodium, potassium, ammonium, and alkylolammonium salts of higher fatty acids containing from about 8 to about 24 carbon atoms, and preferably from about 12 to about 18 carbon atoms. Soaps can be made by direct saponification of fats and oils or by the neutralization of free fatty acids. Particularly useful are the sodium and/or potassium salts of the mixtures of fatty acids derived from coconut oil and tallow, i.e., sodium and/or potassium tallow and/or coconut soap. If high sudsing is desired, the branched-chain C₁₀ - C₁₆ soaps can be used.

Additional anionic surfactants which suitable for use herein include the water-soluble salts, preferably the alkali metal, ammonium and/or alkylolammonium salts, of organic sulfuric reaction products having in their molecular structure an alkyl group containing from about 10 to about 20 carbon atoms and a sulfonic acid or sulfuric acid ester group. (Included in the term "alkyl" is the alkyl portion of acyl groups.) Examples of this group of synthetic surfactants are a) the sodium, potassium and/or ethanolamine alkyl sulfates, especially those obtained by sulfating the higher alcohols (C₈ - C₁₈ carbon atoms) such as those produced by reducing the glycerides of tallow or coconut oil, including primary, branched-chain, and/or random C₁₀ - C₂₀ alkyl sulfates ("AS") [Such alkyl sulfates include the C₁₀ - C₁₈ secondary (2,3) alkyl sulfates of the formula CH₃(CH₂)ₓ (CHOSO₃⁻M⁺) CH₃ and CH₃(CH₂)ₓ (CHOSO₃⁻M⁺) CH₂CH₃ where x and (y+1) are integers of at least about 7, preferably at least about 9, and M is a water-solubilizing cation and/or, especially, sodium; unsaturated sulfates such as oleyl sulfate]; b) the sodium, potassium and ethanolamine alkyl polyethoxylate sulfates, e.g., the C₁₀ - C₂₂ alkyl alkoxy sulfates ("AExS") particularly those in which the alkyl group contains from 10 to 18, preferably from 12 to 18 carbon atoms, and wherein the polyethoxylate chain contains from 1 to 15, preferably 1 to 7 ethoxylate moieties; and c) the sodium and potassium alkylbenzene sulfonates in which the alkyl group contains from about 9 to about 18 carbon atoms, in straight chain or branched chain configuration. Other nonlimiting examples of surfactants useful herein include C₁₀ - C₁₈ alkyl alkoxy carboxylates (especially the EO 1-5 ethoxycarboxylates), the C₁₀ -C₁₈ glycerol ethers, the C₁₀ - C₁₈ alkyl polyglycosides and their corresponding sulfated polyglycosides, and C₁₂ - C₁₈ alpha-sulfonated fatty acid esters. Especially valuable are linear straight chain alkylbenzene sulfonates in which the average number of carbon atoms in the alkyl group is from about 11 to 13, abbreviated as C₁₁₋₁₃ LAS.

The conventional nonionic surfactants such as the C₁₀ - C₁₈ alkyl ethoxylates ("AE") including the so-called narrow peaked alkyl ethoxylates and C₆ - C₁₂ alkyl phenol alkoxylates (especially ethoxylates and mixed ethoxalates/propoxalates), can be used.

Additional suitable nonionic surfactants include polyhydroxy fatty acid amides. Examples are N-methyl N-1-deoxyglucityl cocoamide, N-methyl N-1-deoxyglucityl oleamide, C₁₀ - C₁₈ N-(3-methoxypropyl) glucamide, and the C₁₂ - C₁₈ N-methylglucamides. See WO 92/06154, incorporated herein in its entirety. The N-propyl through N-hexyl C₁₂ - C₁₈ glucamides can be used for low sudsing.

Mixtures of anionic and nonionic surfactants are especially useful.

If desired, the conventional amphoteric surfactants such as the C₁₂ - C₁₈ betaines and sulfobetaines ("sultaines"), C₁₀ - C₁₈ amine oxides, and the like, can also be included in the overall compositions. Other conventional useful surfactants are listed in standard texts.

The C₁₀ - C₁₈ alkyl alkoxy sulfates ("AExS"; especially EO 1-7 ethoxy sulfates) and C₁₂ - C₁₈ alkyl ethoxylates ("AE") are the most preferred for the detergents described herein.

### B. Detergency Builders

Detergency builders can optionally be included in the compositions herein to assist in controlling mineral hardness. Inorganic as well as organic builders can be used. Builders are typically used in fabric laundering compositions to assist in the removal of particulate soils.

The level of builder can vary widely depending upon the end use of the composition and its desired physical form. Granular formulations typically comprise from about 10% to about 80%, more typically from about 15% to about 50% by weight, of the detergent builder. Lower or higher levels of builder, however, are not meant to be excluded.

Inorganic P-containing detergent builders include, but are not limited to, the alkali metal, ammonium and alkanolammonium salts of polyphosphates (exemplified by the tripolyphosphates, pyrophosphates, and glassy polymeric meta-phosphates) and/or phosphonates. In situations where phosphorus-based builders can be used, and especially in the formulation of bars used for hand-laundering operations, the various alkali metal phosphates such as the well-known sodium and/or potassium tripolyphosphates, pyrophosphates and/or orthophosphates can be used. Phosphonate builders such as ethane-I-hydroxy-1,1-diphosphonate and other known phosphonates can also be used. However, non-phosphate builders are required in some locales.

Examples of suitable nonphosphorus, inorganic builders include the silicates, borates phytic acid, carbonates (including bicarbonates and sesquicarbonates), sulfates, and aluminosilicates. Particularly preferred are sodium and potassium carbonate, bicarbonate, sesquicarbonate, tetraborate decahydrate, and silicates having a weight ratio of SiO₂ to alkali metal oxide of from about 0.5 to about 4.0, preferably from about 1.0 to about 2.4. Examples of silicate builders are the alkali metal silicates, particularly those having a SiO₂ : Na₂O ratio in the range 1.6:1 to 3.2:1. Also, crystalline layered silicates such as those discussed in US 4,605,509 are suitable for use in the detergent composition of the invention. Other layered sodium silicates are described in US 4,664,839, NaSKS-6 is the trademark for a crystalline layered silicate marketed by Hoechst (commonly abbreviated herein as "SKS-6"). Unlike zeolite builders, the Na SKS-6 silicate builder does not contain aluminum. NaSKS-6 has the delta-Na₂SiO₅ morphology form of layered silicate. SKS-6 is a highly preferred layered silicate for use herein, but other layered silicates can also be used. Various other layered silicates include NaSKS-5, NaSKS-7 and NaSKS-11, as the alpha, beta and gamma forms. Other silicates can also be useful such as for example magnesium silicate, which can serve as a crispening agent in granular formulations, as a stabilizing agent for oxygen bleaches, and as a component of suds control systems.

Examples of carbonate builders are the alkaline earth and alkali metal carbonates as disclosed in DE 2,321,001.

Aluminosilicate builders are useful in the present invention. Aluminosilicate builders are of great importance in most currently marketed heavy duty granular detergent compositions, and can also be a significant builder ingredient in liquid detergent formulations.

Useful aluminosilicate ion exchange materials are commercially available. These aluminosilicates can be crystalline or amorphous in structure and can be naturally-occurring aluminosilicates or synthetically derived. A method for producing aluminosilicate ion exchange materials is disclosed in US 3,985,669 and incorporated herein in its entirety. Preferred synthetic crystalline aluminosilicate ion exchange materials useful herein are available under the designations Zeolite A, Zeolite P (B), Zeolite MAP and Zeolite X. In an especially preferred embodiment, the crystalline aluminosilicate ion exchange material is Zeolite A. Dehydrated zeolites can also be used herein. Preferably, the aluminosilicate has a particle size of about 0.1-10 microns in diameter.

Water-soluble, nonphosphorus organic builders useful herein include the various alkali metal, ammonium and/or substituted ammonium polyacetates, carboxylates, polycarboxylates and polyhydroxy sulfonates. A wide variety of polycarboxylate compounds are suitable. As used herein, "polycarboxylate" refers to compounds having a plurality of carboxylate groups, preferably at least 3 carboxylates. Polycarboxylate builders can generally be added to the composition in acid form, but can also be added in the form of a neutralized salt. When utilized in salt form, alkali metals, such as sodium, potassium, and lithium, or alkanolammonium salts are preferred.

Other useful detergency builders include the ether hydroxypolycarboxylates, copolymers of maleic anhydride with ethylene or vinyl methyl ether, 1,3,5-trihydroxy benzene-2,4,6-trisulphonic acid, and carboxymethyloxysuccinic acid, the various alkali metal, ammonium and substituted ammonium salts of polyacetic acids such as ethylenediamine tetraacetic acid and nitrilotriacetic acid, as well as polycarboxylates such as mellitic acid, succinic acid, oxydisuccinic acid, polymaleic acid, benzene 1,3,5-tricarboxylic acid, carboxymethyloxysuccinic acid, and soluble salts thereof.

Citrate builders, e.g., citric acid and soluble salts thereof (particularly sodium salt), are polycarboxylate builders of particular importance for heavy duty liquid detergent formulations due to their availability from renewable resources and their biodegradability. Citrates can also be used in granular compositions, especially in combination with zeolite and/or layered silicate builders. Oxydisuccinates are also especially useful in such compositions and combinations.

Also suitable in the detergent compositions of the present invention are the 3,3-dicarboxy-4-oxa-1,6-hexanedioates and related compounds. Useful succinic acid builders include the C₅ - C₂₀ alkyl and alkenyl succinic acids and salts thereof. A particularly preferred compound of this type is dodecenylsuccinic acid. Specific examples of succinate builders include: laurylsuccinate, myristylsuccinate, palmitylsuccinate, 2-dodecenylsuccinate (preferred), 2-pentadecenylsuccinate, and the like. Laurylsuccinates are the preferred builders of this group.

Fatty acids, e.g., C₁₂ - C₁₈ monocarboxylic acids, can also be incorporated into the compositions alone, or in combination with the aforesaid builders, especially citrate and/or the succinate builders, to provide additional builder activity. Such use of fatty acids will generally result in a diminution of sudsing, which should be taken into account by the formulator.

### C. Optional Ingredients

The detergent compositions herein can optionally include one or more other detergent adjunct materials or other materials for assisting or enhancing cleaning performance, treatment of the substrate to be cleaned, or to modify the aesthetics of the detergent composition (e.g., colorants, dyes, etc.). The following are illustrative examples of such adjunct materials.

### 1. Cellulase Enzyme

Cellulase enzymes optionally used in the instant detergent composition are preferably incorporated, when present, at levels sufficient to provide up to about 5 mg by weight, more preferably about 0.01 mg to about 3 mg, of active enzyme per gram of the composition. Unless stated otherwise, the compositions herein preferably comprise from about 0.001 % to about 5%, preferably 0.01%-1% by weight of a commercial enzyme preparation.

The cellulase suitable for the present invention include both bacterial or fungal cellulase. Preferably, they will have a pH optimum of between 5 and 9.5. Suitable cellulases are fungal cellulase produced from Humicola insolens and Humicola strain DSM1800 or a cellulase 212-producing fungus belonging to the genus Aeromonas, and cellulase extracted from the hepatopancreas of a marine mollusk (Dolabella Auricula Solander), suitable cellulases are also disclosed in GB 2,075,028. In addition, cellulase especially suitable for use herein are disclosed in WO 92/13057. Most preferably, the cellulases used in the instant detergent compositions are purchased commercially from NOVO Industries A/S under the product names CAREZYMEO and CELLUZYMEO.

### 2. Other Enzymes

Additional enzymes can be included in the detergent compositions herein for a wide variety of fabric laundering purposes, including removal of protein-based, carbohydrate-based, or triglyceride-based stains, for example, and for the prevention of refugee dye transfer, and for fabric restoration. The additional enzymes to be incorporated include proteases, amylases, lipases, and peroxidases, as well as mixtures thereof. Other types of enzymes can also be included. They can be of any suitable origin, such as vegetable, animal, bacterial, fungal and yeast origin. However, their choice is governed by several factors such as pH-activity and/or stability optima, thermostability, stability versus active detergents, builders as well as their potential to cause malodors during use. In this respect bacterial or fungal enzymes are preferred, such as bacterial amylases and proteases.

Enzymes are normally incorporated at levels sufficient to provide up to about 5 mg by weight, more typically about 0.01 mg to about 3 mg, of active enzyme per gram of the composition. Stated otherwise, the compositions herein will typically comprise from about 0.001 % to about 5%, preferably 0.01%-1% by weight of a commercial enzyme preparation. Protease enzymes are usually present in such commercial preparations at levels sufficient to provide from 0.005 to 0.1 Anson units (AU) of activity per gram of composition.

Suitable examples of proteases are the subtilisins which are obtained from particular strains of B. subtilis and B. licheniforms. Another suitable protease is obtained from a strain of Bacillus, having maximum activity throughout the pH range of 8-12, developed and sold by Novo Industries A/S under the registered trade name ESPERASE®. The preparation of this enzyme and analogous enzymes is described in GB 1,243,784 of Novo. Proteolytic enzymes suitable for removing protein-based stains that are commercially available include those sold under the trade names ALCALASE® and SAVINASE® by Novo Industries A/S and MAXATASE® by International Bio-Synthetics, Inc.. Other proteases include Protease A; Protease B and proteases made by Genencor International, Inc., according to US 5,204,015 and US 5,244,791.

Amylases include, for example, alpha-amylases like RAPIDASE®, International Bio-Synthetics, Inc. and TERMAMYL®, Novo Industries.

Suitable lipase enzymes for detergent usage include those produced by microorganisms of the Pseudomonas group, such as Pseudomonas stutzeri ATCC 19154. This lipase is available from Amano Pharmaceutical Co. Ltd., under the trade name Lipase P "Amano". Other commercial lipases include Amano-CES, lipases ex Chromobacter viscosum, e.g. Chromobacter viscosum var. lipolyticum NRRLB 3673, commercially available from Toyo Jozo Co., and further Chromobacter viscosum lipases from U.S. Biochemical Corp. and Disoynth Co., and lipases ex Pseudomonas gladioli. The LIPOLASE® enzyme derived from Humicola lanuginosa (commercially available from Novo Industries A/S) is a preferred lipase for use herein.

Peroxidase enzymes are used in combination with oxygen sources, e.g., percarbonate, perborate, persulfate, hydrogen peroxide, etc. They are used for "solution bleaching," i.e. to prevent transfer of dyes or pigments removed from substrates during wash operations to other substrates in the wash solution. Peroxidase enzymes are known in the art, and include, for example, horseradish peroxidase, ligninase, and haloperoxidase such as chloro- and bromo-peroxidase. Peroxidase-containing detergent compositions are disclosed, for example, in WO 89/099813.

### 3. Enzyme Stabilizers

The enzymes employed herein are stabilized by the presence of water-soluble sources of calcium and/or magnesium ions in the finished detergent compositions which provide such ions to the enzymes. (Calcium ions are generally somewhat more effective than magnesium ions and are preferred herein if only one type of cation is being used.) Additional stability can be provided by the presence of various other art-disclosed stabilizers, especially borate species, see US 4,537,706, incorporated herein in its entirety. Typical detergents, especially liquids, will comprise from about 1 to about 30, preferably from about 2 to about 20, more preferably from about 5 to about 15, and most preferably from about 8 to about 12, millimoles of calcium ion per liter of finished composition. In solid detergent compositions the formulation can include a sufficient quantity of a water-soluble calcium ion source to provide such amounts in the laundry liquor. In the alternative, natural water hardness can suffice.

It is to be understood that the foregoing levels of calcium and/or magnesium ions are sufficient to provide enzyme stability. More calcium and/or magnesium ions can be added to the compositions to provide an additional measure of grease removal performance. Accordingly, as a general proposition the compositions herein will typically comprise from about 0.05% to about 2% by weight of a water-soluble source of calcium or magnesium ions, or both. The amount can vary, of course, with the amount and type of enzyme employed in the composition.

The compositions herein can also optionally, but preferably, contain various additional stabilizers, especially borate-type stabilizers. Typically, such stabilizers will be used at levels in the compositions from about 0.25% to about 10%, preferably from about 0.5% to about 5%, more preferably from about 0.75% to about 3%, by weight of boric acid or other borate compound capable of forming boric acid in the composition (calculated on the basis of boric acid). Boric acid is preferred, although other compounds such as boric oxide, borax and other alkali metal borates (e.g., sodium ortho-, meta- and pyroborate, and sodium pentaborate) are suitable. Substituted boric acids (e.g., phenylboronic acid, butane boronic acid, and p-bromo phenylboronic acid) can also be used in place of boric acid.

### 4. Bleaching Compounds, Bleaching Agents and Bleach Activators

The detergent compositions herein can optionally contain bleaching agents or bleaching compositions containing a bleaching agent and one or more bleach activators. When present, bleaching agents will typically be at levels of from about 1% to about 30%, more typically from about 5% to about 20%, of the detergent composition, especially for fabric laundering. If present, the amount of bleach activators will typically be from about 0.1 % to about 60%, more typically from about 0.5% to about 40% of the bleaching composition comprising the bleaching agent-plus-bleach activator.

The bleaching agents used herein can be any of the bleaching agents useful for detergent compositions in textile cleaning, hard surface cleaning, or other cleaning purposes that are now known or become known. These include oxygen bleaches as well as other bleaching agents. Perborate bleaches, e.g., sodium perborate (e.g., mono- or tetrahydrate) can be used herein.

Another category of bleaching agent that can be used without restriction encompasses percarboxylic acid bleaching agents and salts thereof. Suitable examples of this class of agents include magnesium monoperoxyphthalate hexahydrate, the magnesium salt of meta-chloro perbenzoic acid, 4-nonylamino-4-oxoperoxybutyric acid and diperoxydodecanedioic acid.

Peroxygen bleaching agents can also be used. Suitable peroxygen bleaching compounds include sodium carbonate peroxyhydrate and equivalent "percarbonate" bleaches, sodium pyrophosphate peroxyhydrate, urea peroxyhydrate, and sodium peroxide. Persulfate bleach (e.g., OXONEO®, manufactured commercially by DuPont) can also be used.

A preferred percarbonate bleach comprises dry particles having an average particle size in the range from about 500 micrometers to about 1,000 micrometers, not more than about 10% by weight of said particles being smaller than about 200 micrometers and not more than about 10% by weight of said particles being larger than about 1,250 micrometers. Optionally, the percarbonate can be coated with silicate, borate or water-soluble surfactants. Percarbonate is available from various commercial sources.

Mixtures of bleaching agents can also be used.

Peroxygen bleaching agents, the perborates, the percarbonates, etc., are preferably combined with bleach activators, which lead to the in situ production in aqueous solution (i.e., during the washing process) of the peroxy acid corresponding to the bleach activator. The nonanoyloxybenzene sulfonate (NOBS) and tetraacetyl ethylene diamine (TAED) activators are typical, and mixtures thereof can also be used.

Preferred amido-derived bleach activators include (6-octanamido-caproyl)oxybenzenesulfonate, (6-nonanamidocaproyl)oxybenzenesulfonate, (6-decanamido-caproyl)oxybenzenesulfonate, and mixtures thereof.

Another class of bleach activators comprises the benzoxazin-type activators disclosed in US 4,966,723.

Highly preferred lactam activators include benzoyl caprolactam, octanoyl caprolactam, 3,5,5-trimethylhexanoyl caprolactam, nonanoyl caprolactam, decanoyl caprolactam, undecenoyl caprolactam, benzoyl valerolactam, octanoyl valerolactam, decanoyl valerolactam, undecenoyl valerolactam, nonanoyl valerolactam, 3,5,5-trimethylhexanoyl valerolactam and mixtures thereof, optionally adsorbed into solid carriers, e.g acyl caprolactams, preferably benzoyl caprolactam, adsorbed into sodium perborate.

Bleaching agents other than oxygen bleaching agents are also known in the art and can be utilized herein. One type of non-oxygen bleaching agent of particular interest includes photoactivated bleaching agents such as the sulfonated zinc and/or aluminum phthalocyanines. If used, detergent compositions will typically contain from about 0.025% to about 1.25%, by weight, of such bleaches, especially sulfonate zinc phthalocyanine.

If desired, the bleaching compounds can be catalyzed by means of a manganese compound. Such manganese-based catalysts are well known in the art and include Mn^{IV}₂ (u-O)₃ (1,4,7-trimethyl-1,4,7-triazacyclononane)₂ (PF₆)₂, Mn^{III}₂ (u-O)₁ (u-OAc)₂ (1,4,7-trimethyl-1,4,7-triazacyclononane)₂(ClO₄)₂, Mn^{IV}₄ (u-O)₆ (1,4,7-triazacyclononane)₄ (CIO₄)₄, Mn^{III}Mn^{IV}₄ (u-O)₁ (u-OAc)₂(1,4,7-trimethyl-1,4,7-triazacyclononane)₂ (ClO₄)₃, Mn^{IV} (1,4,7-trimethyl-1,4,7-triazacyclononane)-(OCH₃)₃(PF₆), and mixtures thereof.

As a practical matter, and not by way of limitation, the compositions and processes herein can be adjusted to provide on the order of at least one part per ten million of the active bleach catalyst species in the aqueous washing liquor, and will preferably provide from about 0.1 ppm to about 700 ppm, more preferably from about 1 ppm to about 500 ppm, of the catalyst species in the laundry liquor.

### 5. Polymeric Soil Release Agent

Any polymeric soil release agent known to those skilled in the art can optionally be employed in the detergent compositions and processes of this invention. Polymeric soil release agents are characterized by having both hydrophilic segments, to hydrophilize the surface of hydrophobic fibers, such as polyester and nylon, and hydrophobic segments, to deposit upon hydrophobic fibers and remain adhered thereto through completion of washing and rinsing cycles and, thus, serve as an anchor for the hydrophilic segments. This can enable stains occurring subsequent to treatment with the soil release agent to be more easily cleaned in later washing procedures.

The polymeric soil release agents useful herein especially include those soil release agents having: (a) one or more nonionic hydrophile components consisting essentially of (i) polyoxyethylene segments with a degree of polymerization of at least 2, or (ii) oxypropylene or polyoxypropylene segments with a degree of polymerization of from 2 to 10, wherein said hydrophile segment does not encompass any oxypropylene unit unless it is bonded to adjacent moieties at each end by ether linkages, or (iii) a mixture of oxyalkylene units comprising oxyethylene and from 1 to about 30 oxypropylene units wherein said mixture contains a sufficient amount of oxyethylene units such that the hydrophile component has hydrophilicity great enough to increase the hydrophilicity of conventional polyester synthetic fiber surfaces upon deposit of the soil release agent on such surface, said hydrophile segments preferably comprising at least about 25% oxyethylene units and more preferably, especially for such components having about 20 to 30 oxypropylene units, at least about 50% oxyethylene units; or (b) one or more hydrophobe components comprising (i) C₃ oxyalkylene terephthalate segments, wherein, if said hydrophobe components also comprise oxyethylene terephthalate, the ratio of oxyethylene terephthalate: C₃ oxyalkylene terephthalate units is about 2:1 or lower, (ii) C₄ - C₆ alkylene or oxy C₄ - C₆ alkylene segments, or mixtures therein, (iii) poly (vinyl ester) segments, preferably polyvinyl acetate), having a degree of polymerization of at least 2, or (iv) C₁ - C₄ alkyl ether or C₄ hydroxyalkyl ether substituents, or mixtures therein, wherein said substituents are present in the form of C₁ - C₄ alkyl ether or C₄ hydroxyalkyl ether cellulose derivatives, or mixtures therein, and such cellulose derivatives are amphiphilic, whereby they have a sufficient level of C₁ - C₄ alkyl ether and/or C₄ hydroxyalkyl ether units to deposit upon conventional polyester synthetic fiber surfaces and retain a sufficient level of hydroxyls, once adhered to such conventional synthetic fiber surface, to increase fiber surface hydrophilicity, or a combination of (a) and (b).

Typically, the polyoxyethylene segments of (a) (i) will have a degree of polymerization of from about 200, although higher levels can be used, preferably from 3 to about 150, more preferably from 6 to about 100. Suitable oxy C₄ - C₆ alkylene hydrophobe segments include, but are not limited to, end-caps of polymeric soil release agents.

Polymeric soil release agents useful in the present invention also include cellulosic derivatives such as hydroxyether cellulosic polymers, copolymeric blocks of ethylene terephthalate or propylene terephthalate with polyethylene oxide or polypropylene oxide terephthalate, and the like. Such agents are commercially available and include hydroxyethers of cellulose such as METHOCEL® (Dow). Cellulosic soil release agents for use herein also include those selected from the group consisting of C₁ - C₄ alkyl and C₄ hydroxyalkyl cellulose.

Soil release agents characterized by poly(vinyl ester) hydrophobe segments include graft copolymers of poly(vinyl ester), e.g., C₁ - C₆ vinyl esters, preferably poly(vinyl acetate) grafted onto polyalkylene oxide backbones, such as polyethylene oxide backbones, see EP 0 219 048, incorporated herein in its entirety. Commercially available soil release agents of this kind include the SOKALAN® type of material, e.g., SOKALAN® HP-22, available from BASF.

One type of preferred soil release agent is a copolymer having random blocks of ethylene terephthalate and polyethylene oxide (PEO) terephthalate. The molecular weight of this polymeric soil release agent preferably is in the range of from about 25,000 to about 55,000.

Another preferred polymeric soil release agent is a polyester with repeat units of ethylene terephthalate units contains 10-15% by weight of ethylene terephthalate units together with 90-80% by weight of polyoxyethylene terephthalate units, derived from a polyoxyethylene glycol of average molecular weight 300-5,000. Examples of this polymer include the commercially available material ZELCON® 5126 (from DuPont) and MILEASE® T (from ICI).

Another preferred polymeric soil release agent is a sulfonated product of a substantially linear ester oligomer comprised of an oligomeric ester backbone of terephthaloyl and oxyalkyleneoxy repeat units and terminal moieties covalently attached to the backbone. These soil release agents are described fully in US 4,968,451. Other suitable polymeric soil release agents include the terephthalate polyesters of US 4,711,730, the anionic end-capped oligomeric esters of US 4,721,580, the block polyester oligomeric compounds of US 4,702,857, and anionic, especially sulfoaroyl, end-capped terephthalate esters of US 4,877,896 .

Still another preferred soil release agent is an oligomer with repeat units of terephthaloyl units, sulfoisoterephthaloyl units, oxyethyleneoxy and oxy-1,2-propylene units. The repeat units form the backbone of the oligomer and are preferably terminated with modified isethionate end-caps. A particularly preferred soil release agent of this type comprises about one sulfoisophthaloyl unit, 5 terephthaloyl units, oxyethyleneoxy and oxy-1,2-propyleneoxy units in a ratio of from about 1.7 to about 1.8, and two end-cap units of sodium 2-(2-hydroxyethoxy)-ethanesulfonate. Said soil release agent also comprises from about 0.5% to about 20%, by weight of the oligomer, of a crystalline-reducing stabilizer, preferably selected from the group consisting of xylene sulfonate, cumene sulfonate, toluene sulfonate, and mixtures thereof.

If utilized, soil release agents will generally comprise from about 0.01% to about 10.0%, by weight, of the detergent compositions herein, typically from about 0.1% to about 5%, preferably from about 0.2% to about 3.0%.

### 6. Chelating Agents

The detergent compositions herein can also optionally contain one or more iron and/or manganese chelating agents. Such chelating agents can be selected from the group consisting of amino carboxylates, amino phosphonates, polyfunctionally-substituted aromatic chelating agents and mixtures therein, all as hereinafter defined. Without intending to be bound by theory, it is believed that the benefit of these materials is due in part to their exceptional ability to remove iron and manganese ions from washing solutions by formation of soluble chelates. It is understood that some of the detergent builders described hereinbefore can function as chelating agents and is such detergent builder is present in a sufficient quantity, it can provide both functions.

Amino carboxylates useful as optional chelating agents include ethylenediaminetetracetates, N-hydroxyethylethylenediaminetriacetates, nitrilotriacetates, ethylenediamine tetraproprionates, triethylenetetraaminehexacetates, diethylenetriaminepentaacetates, and ethanoldiglycines, alkali metal, ammonium, and substituted ammonium salts therein and mixtures therein.

Amino phosphonates are also suitable for use as chelating agents in the compositions of the invention when at lease low levels of total phosphorus are permitted in detergent compositions, and include ethylenediaminetetrakis (methylenephosphonates) as DEQUEST. Preferred, these amino phosphonates to not contain alkyl or alkenyl groups with more than about 6 carbon atoms.

Polyfunctionally-substituted aromatic chelating agents are also useful in the compositions herein. Preferred compounds of this type in acid form are dihydroxydisulfobenzenes such as 1,2-dihydroxy-3,5-disulfobenzene.

A preferred biodegradable chelator for use herein is ethylenediamine disuccinate ("EDDS"), especially the [S,S] isomer.

If utilized, these chelating agents will generally comprise from about 0.1 % to about 10% by weight of the detergent compositions herein. More preferably, if utilized, the chelating agents will comprise from about 0.1 % to about 3.0% by weight of such compositions.

### 7. Clay Soil Removal/Anti-redeposition Agents

The detergent compositions of the present invention can also optionally contain water-soluble ethoxylated amines having clay soil removal and antiredeposition properties. Granular detergent compositions which contain these compounds typically contain from about 0.01% to about 10.0% by weight of the water-soluble ethoxylates amines; liquid detergent compositions typically contain about 0.01 % to about 5%.

The most preferred soil release and anti-redeposition agent is ethoxylated tetraethylenepentamine. Exemplary ethoxylated amines are further described in US 4,597,898. Other groups of preferred clay soil removal-antiredeposition agents are the cationic compounds disclosed in EP 0 111 965, the ethoxylated amine polymers disclosed in EP 0 111 984, the zwitterionic polymers disclosed in EP 0 112 592, and the amine oxides disclosed in US 4,548,744. Another type of preferred antiredeposition agent includes the carboxy methyl cellulose (CMC) materials. These materials are well known in the art.

### 8. Polymeric Dispersing Agents

Polymeric dispersing agents can advantageously be utilized at levels from about 0.1 % to about 7%, by weight, in the detergent compositions herein, especially in the presence of zeolite and/or layered silicate builders. Suitable polymeric dispersing agents include polymeric polycarboxylates and polyethylene glycols, although others known in the art can also be used. It is believed, though it is not intended to be limited by theory, that polymeric dispersing agents enhance overall detergent builder performance, when used in combination with other builders (including lower molecular weight polycarboxylates) by crystal growth inhibition, particulate soil release peptization, and anti-redeposition.

Polymeric polycarboxylate materials can be prepared by polymerizing or copolymerizing suitable unsaturated monomers, preferably in their acid form. Unsaturated monomeric acids that can be polymerized to form suitable polymeric polycarboxylates include acrylic acid, maleic acid (or maleic anhydride), fumaric acid, itaconic acid, aconitic acid, mesaconic acid, citraconic acid and methylenemalonic acid. The presence in the polymeric polycarboxylates herein or monomeric segments, containing no carboxylate radicals such as vinylmethyl ether, styrene, ethylene, etc. is suitable provided that such segments do not constitute more than about 40% by weight.

Particularly suitable polymeric polycarboxylates can be derived from acrylic acid. Such acrylic acid-based polymers which are useful herein are the water-soluble salts of polymerized acrylic acid. The average molecular weight of such polymers in the acid form preferably ranges from about 2,000 to 10,000, more preferably from about 4,000 to 7,000 and most preferably from about 4,000 to 5,000. Water-soluble salts of such acrylic acid polymers can include, for example, the alkali metal, ammonium and substituted ammonium salts. Soluble polymers of this type are known materials. Use of polyacrylates of this type in detergent compositions has been disclosed, for example US 3,308,067.

Acrylic/maleic-based copolymers can also be used as a preferred component of the dispersing/anti-redeposition agent. Such materials include the water-soluble salts of copolymers of acrylic acid and maleic acid. The average molecular weight of such copolymers in the acid form preferably ranges from about 2,000 to 100,000, more preferably from about 5,000 to 75,000, most preferably from about 7,000 to 65,000. The ratio of acrylate to maleate segments in such copolymers will generally range from about 30:1 to about 1:1, more preferably from about 10:1 to 2:1. Water-soluble salts of such acrylic acid/maleic acid copolymers can include, for example, the alkali metal, ammonium and substituted ammonium salts. Soluble acrylate/maleate copolymers of this type are known materials which are described in EP 0 193 360, which also describes such polymers comprising hydroxypropylacrylate. Still other useful dispersing agents include the maleic/acrylic/vinyl alcohol terpolymers, for example, a 45/45/10 terpolymer of acrylic/maleic/vinyl alcohol.

Another polymeric material which can be included is polyethylene glycol (PEG). PEG can exhibit dispersing agent performance as well as act as a clay soil removal-antiredeposition agent. Typical molecular weight ranges for these purposes range from about 500 to about 100,000, preferably from about 1,000 to about 50,000, more preferably from about 1,500 to about 10,000.

Polyaspartate and polyglutamate dispersing agents can also be used, especially in conjunction with zeolite builders. Dispersing agents such as polyaspartate preferably have a molecular weight (avg.) of about 10,000.

### 9. Brighteners

Any optical brighteners or other brightening or whitening agents known in the art can be incorporated at levels typically from about 0.05% to about 1.2%, by weight, into the detergent compositions herein. Commercial optical brighteners which can be useful in the present invention can be classified into subgroups, which include, but are not necessarily limited to, derivatives of stilbene, pyrazoline, coumarin, carboxylic acid, methinecyanines, dibenzothiphene-5,5-dioxide, azoles, 5- and 6-membered-ring heterocycles, and other miscellaneous agents.

Preferred brighteners include the PHORWHITE® series of brighteners from Verona. Other brighteners disclosed in this reference include: Tinopal® UNPA, Tinopal CBS and Tinopal 5BM; available from Ciba-Geigy; Artic White® CC and Artic White CWD, available from Hilton-Davis; the 2-(4-stryl-phenyl)-2H-napthol [1,2-d]triazoles; 4,4'-bis-(1,2,3-triazol-2-yl)-stilbenes; 4,4'-bis(stryl)bisphenyls; and the aminocoumarins. Specific examples of these brighteners include 4-methyl-7-diethyl-amino coumarin; 1,2-bis(-venzimidazol-2-yl)ethylene; 1,3-diphenyl-phrazolines; 2,5-bis(benzoxazol-2-yl)thiophene; 2-stryl-napth- [1,2-d] oxazole; and 2-(stilbene-4-yl)-2H-naphtho- [1,2-d]triazole. Anionic brighteners are preferred herein.

### 10. Dye Transfer Inhibiting Agents

The detergent compositions of the present invention can also include one or more materials effective for inhibiting the transfer of dyes from one fabric to another during the cleaning process. Generally, such dye transfer inhibiting agents include polyvinyl pyrrolidone polymers, polyamine N-oxide polymers, copolymers of N-vinylpyrrolidone and N-vinylimidazole, manganese phthalocyanine, peroxidases, and mixtures thereof. If used, these agents typically comprise from about 0.01% to about 10% by weight of the composition, preferably from about 0.01% to about 5%, and more preferably from about 0.05% to about 2%.

More specifically, the polyamine N-oxide polymers preferred for use herein are described in US 6,491,728.

Any polymer backbone can be used as long as the amine oxide polymer formed is water-soluble and has dye transfer inhibiting properties. Examples of suitable polymeric backbones are polyvinyls, polyalkylenes, polyesters, polyethers, polyamide, polyimides, polyacrylates and mixtures thereof. These polymers include random or block copolymers where one monomer type is an amine N-oxide and the other monomer type is an N-oxide. The amine N-oxide polymers typically have a ratio of amine to the amine N-oxide of 10:1 to 1:1,000,000. However, the number of amine oxide groups present in the polyamine oxide polymer can be varied by appropriate copolymerization or by an appropriate degree of N-oxidation. The polyamine oxides can be obtained in almost any degree of polymerization. Typically, the average molecular weight is within the range of 500 to 1,000,000; more preferred 1,000 to 500,000; most preferred 5,000 to 100,000. This preferred class of materials can be referred to as "PVNO".

The most preferred polyamine N-oxide useful in the detergent compositions herein is poly(4-vinylpyridine-N-oxide) which as an average molecular weight of about 50,000 and an amine to amine N-oxide ratio of about 1:4.

Copolymers of N-vinylpyrrolidone and N-vinylimidazole polymers (referred to as a class as "PVPVI") are also preferred for use herein. Preferably the PVPVI has an average molecular weight range from 5,000 to 1,000,000, more preferably from 5,000 to 200,000, and most preferably from 10,000 to 20,000. The PVPVI copolymers typically have a molar ratio of N-vinylimidazole to N-vinylpyrrolidone from 1:1 to 0.2:1, more preferably from 0.8:1 to 0.3:1, most preferably from 0.6:1 to 0.4:1. These copolymers can be either linear or branched.

The present invention compositions also can employ a polyvinylpyrrolidone ("PVP") having an average molecular weight of from about 5,000 to about 400,000, preferably from about 5,000 to about 200,000, and more preferably from about 5,000 to about 50,000. PVP's are known to persons skilled in the detergent field. Compositions containing PVP can also contain polyethylene glycol ("PEG") having an average molecular weight from about 500 to about 100,000, preferably from about 1,000 to about 10,000. Preferably, the ratio of PEG to PVP on a ppm basis delivered in wash solutions is from about 2:1 to about 50:1, and more preferably from about 3:1 to about 10:1.

The detergent compositions herein can also optionally contain from about 0.005% to 5% by weight of certain types of hydrophilic optical brighteners which also provide a dye transfer inhibition action. If used, the compositions herein will preferably comprise from about 0.01 % to 1% by weight of such optical brighteners.

One preferred brightener is 4,4',-bis[(4-anilino-6-(N-2-bis-hydroxyethyl)-s-triazine-2-yl)amino]-2,2'-stilbenedisulfonic acid and disodium salt. This particular brightener species is commercially marketed under the trade name Tinopal-UNPA-GX® by Ciba-Geigy Corporation. Tinopal-UNPA-GX is the preferred hydrophilic optical brightener useful in the detergent compositions herein.

Another preferred brightener is 4,4'-bis[(4-anilino-6-(N-2-hydroxyethyl-N-methylamino)-s-triazine-2-yl)amino]2,2'-stilbenedisulfonic acid disodium salt. This particular brightener species is commercially marketed under the trade name Tinopal 5BM-GX® by Ciba-Geigy Corporation.

Another preferred brightener brightener is 4,4'-bis[(4-anilino-6-morphilino-s-triazine-2-yl)amino]2,2'-stilbenedisulfonic acid, sodium salt. This particular brightener species is commercially marketed under the trade name Tinopal AMS-GX® by Ciba Geigy Corporation.

The specific optical brightener species selected for use in the present invention provide especially effective dye transfer inhibition performance benefits when used in combination with the selected polymeric dye transfer inhibiting agents hereinbefore described. The combination of such selected polymeric materials (e.g., PVNO and/or PVPVI) with such selected optical brighteners (e.g., Tinopal UNPA-GX, Tinopal 5BM-GX and/or Tinopal AMS-GX) provides significantly better dye transfer inhibition in aqueous wash solutions than does either of these two detergent composition components when used alone. Without being bound by theory, it is believed that such brighteners work this way because they have high affinity for fabrics in the wash solution and therefore deposit relatively quick on these fabrics. The extent to which brighteners deposit on fabrics in the wash solution can be defined by a parameter called the "exhaustion coefficient". The exhaustion coefficient is in general as the ratio of a) the brightener material deposited on fabric to b) the initial brightener concentration in the wash liquor. Brighteners with relatively high exhaustion coefficients are the most suitable for inhibiting dye transfer in the context of the present invention.

Of course, it will be appreciated that other, conventional optical brightener types of compounds can optionally be used in the present compositions to provide conventional fabric "brightness" benefits, rather than a true dye transfer inhibiting effect. Such usage is conventional and well-known to detergent formulations.

### 11. Suds Suppressors

Compounds for reducing or suppressing the formation of suds can be incorporated into the detergent compositions of the present invention. Suds suppression can be of particular importance in the so-called "high concentration cleaning process" and in front-loading European-style washing machines.

A wide variety of materials can be used as suds suppressors, and suds suppressors are well known to those skilled in the art. See, for example, Kirk Othmer Encyclopedia of Chemical Technology, Third Edition, Volume 7, pages 430-447 (John Wiley & Sons, Inc., 1979). One category of suds suppressor of particular interest encompasses monocarboxylic fatty acid and soluble salts therein. The monocarboxylic fatty acids and salts thereof used as suds suppressor typically have hydrocarbyl chains of 10 to about 24 carbon atoms, preferably 12 to 18 carbon atoms. Suitable salts include the alkali metal salts such as sodium, potassium, and lithium salts, and ammonium and alkanolammonium salts.

The detergent compositions herein can also contain non-surfactant suds suppressors. These include, for example: high molecular weight hydrocarbons such as paraffin, fatty acid esters (e.g., fatty acid triglycerides), fatty acid esters of monovalent alcohols, aliphatic C₁₈- C₄₀ ketones (e.g., stearone), etc. Other suds inhibitors include N-alkylated amino triazines such as tri- to hexa-alkylmelamines or di- to tetra-alkyldiamine chlortriazines formed as products of cyanuric chloride with two or three moles of a primary or secondary amine containing 1 to 24 carbon atoms, propylene oxide, and monostearyl phosphates such as monostearyl alcohol phosphate ester and monostearyl di-alkali metal (e.g., K, Na, and Li) phosphates and phosphate esters. The hydrocarbons such as paraffin and haloparaffin can be utilized in liquid form. The liquid hydrocarbons will be liquid at room temperature and atmospheric pressure, and will have a pour point in the range of about -40°C and about 50°C, and a minimum boiling point not less than about 110°C (atmospheric pressure). It is also known to utilize waxy hydrocarbons, preferably having a melting point below about 100°C. Hydrocarbon suds suppressors are known in the art and include aliphatic, alicyclic, aromatic, and heterocyclic saturated or unsaturated hydrocarbons having from about 12 to about 70 carbon atoms. The term "paraffin," as used in this suds suppressor discussion, is intended to include mixtures of true paraffins and cyclic hydrocarbons.

Another preferred category of non-surfactant suds suppressors comprises silicone suds suppressors. This category includes the use of polyorganosiloxane oils, such as polydimethylsiloxane, dispersions or emulsions of polyorganosiloxane oils or resins, and combinations of polyorganosiloxane with silica particles wherein the polyorganosiloxane is chemisorbed or fused onto the silica. Silicone suds suppressors are well known in the art.

Other silicone suds suppressors are disclosed in US 3,455,839, which relates to compositions and processes for defoaming aqueous solutions by incorporating therein small amounts of polydimethylsiloxane fluids.

Mixtures of silicone and silanated silica are described, for instance, in DE 2,124,526. Silicone defoamers and suds controlling agents in granular detergent compositions are disclosed in US 4,652,392.

In the preferred silicone suds suppressor used herein, the solvent for a continuous phase is made up of certain polyethylene glycols or polyethylene-polypropylene glycol copolymers or mixtures thereof (preferred), or polypropylene glycol. The primary silicone suds suppressor is branched/crosslinked and preferably not linear.

The silicone suds suppressor herein preferably comprises polyethylene glycol and a copolymer of polyethylene glycol/polypropylene glycol, all having an average molecular weight of less than about 1,000, preferably between about 100 and 800. The polyethylene glycol and polyethylene/polypropylene copolymers herein have a solubility in water at room temperature of more than about 2 weight %, preferably more than about 5 weight %.

The preferred solvent herein is polyethylene glycol having an average molecular weight of less than about 1,000, more preferably between about 100 and 800, most preferably between 200 and 400, and a copolymer of polyethylene glycol/polypropylene glycol, preferably PPG 200/PEG 300. Preferred is a weight ratio of between about 1:1 and 1:10, most preferably between 1:3 and 1:6, of polyethylene glycol:copolymer of polyethylene-polypropylene glycol.

The preferred silicone suds suppressors used herein do not contain polypropylene glycol, particularly of 4,000 molecular weight. They also preferably do not contain block copolymers of ethylene oxide and propylene oxide, like PLURONIC® L101.

Other suds suppressors useful herein comprise the secondary alcohols (e.g., 2-alkyl alkanols) and mixtures of such alcohols with silicone oils. The secondary alcohols include the C₆ - C₁₆ alkyl alcohols having a C₁ - C₁₆ chain. A preferred alcohol is 2-butyl octanol, which is available from Condea under the trademark ISOFOL® 12. Mixtures of secondary alcohols are available under the trademark ISALCHEM® 123 from Enichem. Mixed suds suppressors typically comprise mixtures of alcohol+silicone at a weight ratio of 1:5 to 5:1.

The compositions herein will generally comprise from 0% to about 5% of suds suppressor. When utilized as suds suppressors, monocarboxylic fatty acids, and salts therein, will be present typically in amounts up to about 5%, by weight, of the detergent composition. Preferably, from about 0.5% to about 3% of fatty monocarboxylate suds suppressor is utilized. Silicone suds suppressors are typically utilized in amounts up to about 2.0%, by weight, of the detergent composition, although higher amounts can be used. This upper limit is practical in nature, due primarily to concern with keeping costs minimized and effectiveness of lower amounts for effectively controlling sudsing. Preferably from about 0.01% to about 1% of silicone suds suppressor is used, more preferably from about 0.25% to about 0.5%. As used herein, these weight percentage values include any silica that can be utilized in combination with polyorganosiloxane, as well as any adjunct materials that can be utilized. Monostearyl phosphate suds suppressors are generally utilized in amounts ranging from about 0.1 % to about 2%, by weight, of the composition. Hydrocarbon suds suppressors are typically utilized in amounts ranging from about 0.01% to about 5.0%, although higher levels can be used. The alcohol suds suppressors are typically used at 0.2%-3% by weight of the finished compositions.

### 12. Fabric Softeners

Various through-the-wash fabric softeners, especially the impalpable smectite clays of US 4,062,647, , as well as other softener clays known in the art, can optionally be used typically at levels of from about 0.5% to about 10% by weight in the present detergent compositions to provide fabric softener benefits concurrently with fabric cleaning. Clay softeners can be used in combination with amine and cationic softeners as disclosed, for example, in US 4,375,416, and US 4,291,071.

### 13. Other Ingredients

A wide variety of other ingredients useful in detergent compositions can be included in the compositions herein, including other active ingredients, carriers, hydrotropes, processing aids, dyes or pigments, solvents for liquid formulations, solid fillers for bar compositions, etc. If high sudsing is desired, suds boosters such as the C₁₀- C₁₆ alkanolamides can be incorporated into the compositions, typically at 1%-10% levels. The C₁₀ - C₁₄ monoethanol and diethanol amides illustrate a typical class of such suds boosters. Use of such suds boosters with high sudsing adjunct surfactants such as the amine oxides, betaines and sultaines noted above is also advantageous. If desired, soluble magnesium salts such as MgCl₂, MgSO₄, and the like, can be added at levels of, typically, 0.1%-2%, to provide additional suds and to enhance grease removal performance.

The detergent compositions herein will preferably be formulated such that, during use in aqueous cleaning operations, the wash water will have a pH of between about 6.5 and about 11, preferably between about 7.5 and 10.5. Laundry products are typically at pH 9-11. Techniques for controlling pH at recommended usage levels include the use of buffers, alkalis, acids, etc., and are well known to those skilled in the art.

### Antiperspirants / Deodorants

Deodorants are inventive preparations, which have antimicrobial activity and which mask, remove, or decrease perspiration odor. Antiperspirants are inventive substances, which have astringent action and inhibit the flow of perspiration.

Antiperspirants inhibit the secretion of sweat and thus remove the bacteria responsible for body odor from the breeding ground. As antiperspirants astringent metal salts are generally used, in particular inorganic and organic metal salts of the elements aluminum, zinc, magnesium, tin and zirconium as well as mixtures of these, wherein in particular halogenides such as aluminum hydroxychloride, zirconyl oxychloride and zirconyl hydroxychloride as well as mixtures of these are used. Frequently these aluminum and zirconium salts and their mixtures are also used in complex form, with propylene glycol, polyethylene glycol or glycerine being used as complexing agents.

In the following one ore more antiperspirants are preferably selected from the group selected of:
aluminium chlorohydrate; aluminium sesquichlorohydrate, aluminium chlorohydrex PG, aluminium dichlorohydrex PG, aluminium sesquichlorohydrex PG, aluminium chlorohydrex PEG, aluminium dichlorohydrex PEG, aluminium sesquichlorohydrex PEG, aluminium chloride (preferably in the form of a 15% by weight (aqueous) solution), aluminium zirconium chlorohydrate, aluminium zirconium trichlorohydrate, aluminium zirconium tetrachlorohydrate, aluminium zirconium pentachlorohydrate, aluminium zirconium octachlorohydrate, aluminium zirconium trichlorohydrex-gly, aluminium zirconium tetrachlorohydrex-gly, aluminium zirconium pentachlorohydrex-gly, aluminium zirconium octachlorohydrex-gly, buffered aluminium sulphate, basic aluminium chloride, zirconium hydroxychloride, zirconium chloride, basic aluminium nitrate, basic aluminium chloride combined with zirconyloxychloride and -hydroxychloride, organic complexes of basic aluminium chlorides and/or zirconium chloride and/or zirconium hydroxychloride.

In turn, preference here is for aluminum or aluminum zirconium complexes with a met-al/anion ratio in the range 0.9:1 to 2.1:1, wherein the anion is preferably selected from the group comprising Cl⁻, Br⁻, I⁻ and/or NO₃⁻, if necessary in combination with additives such as amino acids (preferably glycine) or mono- or polyvalent alcohols. The polyvalent alcohols are preferably di-, tri- or polyols with 3 - 12 C-atoms, with preference here being in turn for glycerine, propylene glycol, diglycerine, tripropylene glycol, sorbitol, 1,2,4-butanetriol or 1,2,6-hexanetriol and mixtures thereof, with particular preference for glycerine and diglycerine. Advantageous monoalcohols are glycol ethers such as monoalkylether or alpha-hydroxy acids such as lactic acid.

In connection with the present invention mixtures of antiperspirants comprising (i) aluminum and/or zirconium and (ii) zinc and/or tin can be used, such as Al/Zr/Zn, Al/Zn, Al/Sn or Al/Sr/Sn, wherein mixtures comprising one or more antiperspirants from among the abovementioned preferred aluminum- and or zirconium-based antiperspirants are in turn preferred.

If necessary the deodorants and antiperspirants according to the invention, apart from the special active ingredients described above, can contain one or more additional substances:
propellant gases, ethanol, propylene glycol, emulsifiers such as aminomethylpropanol, skin care/moisturising agents such as 2-octyldodecanol, isopropylmyristate, isopropylpalmitate, stearamide, sorbitol, glycerine and modified polyethylene- and polypropylenglycols, vitamins and their derivatives (e.g. tocopherol (vitamin E), tocopheryl acetate (vitamin E - acetate) and ascorbic acid (vitamin C)), panthenol, allantoine, plant extracts, such as Aloe Vera and proteins, lustre agents, electrolyte salts such as KCI, NaCl, gelling substances such as hydroxyalkylcelluloses, fatty alcohols, fatty acids, fatty alcohol fatty acid esters, fatty acid glyceryl esters or similar, liquid carriers and solvents such as volatile and non-volatile silicon oils, solid carriers such as talcum, silica gels, and similar; antioxidants and preservatives, UV-light protective filters, cooling agents, additional fragrancing materials, in order to modify odor types, which increase sensory acceptance and/or improve the hedonistic feeling.

The antiperspirant/deodorant compositions of the present invention may be in the form of a solid stick, an aerosol, a pump spray, a roll-on, cream, lotion, or powder. A conventional solid stick generally comprises a wax base into which the antiperspirant salts are incorporated. Roll-ons and lotions are liquid based with various possible liquids serving as the vehicle. Silicones, glycols, emollients, and etc., represent some of the suitable vehicles. A number of nonessential constituents such as suspending agents, drying agents and emollients may be added to enhance cosmetic effects.

In antiperspirant creams of the present invention, the vehicle is a cream. Generally, creams contain oils and light waxes to provide the cream effect. It may also be desired to add nonessential but desirable constituents such as suspending agents, silicones, alcohol, whitening agents, and so forth. Antiperspirant powders are obviously powder based. The vehicle comprises powder constituents such as talc, kaolin, and other similar powder constituents. Other inventive antiperspirant types include pads and gels.

### Fabric Softener Sheets

(see also US 5,348,667)

In preferred embodiments, the present invention encompasses processes for preparing articles of manufacture. These articles are adapted for use to provide unique perfume benefits and to condition (soften) fabrics in an automatic laundry dryer. Such articles are disclosed in US 5,348,667 and the references cited therein.

The preferred fabric softener sheet of the preset invention comprises a Perfume, Perfume Particles, Fabric Conditioning Agents and a Dispensing Means.

Preferred fabric softener sheets comprise or consist of:
(A) a fabric conditioning composition comprising:
   i. from about 30% to about 99% of fabric conditioning, preferably softening, agent; and
   ii. an effective amount, preferably from about 0.5% to about 60%, of free perfume oil and particles according to the present invention, and
(B) a dispensing means which provides for release of an effective amount of said composition to fabrics in an automatic laundry dryer at automatic laundry dryer operating temperatures, e.g., from about 35°C to 115°C.

When the dispensing means is a flexible substrate, e.g., in sheet configuration, the fabric conditioning composition is releasably affixed on the substrate to provide a weight ratio of conditioning composition to dry substrate ranging from about 10:1 to about 0.5:1, preferably from about 5:1 to about 1:1. The invention comprises the method of manufacturing such an article of manufacture utilizing said complex ii. by premixing the complex ii. with at least a portion of the fabric softening agent i. and mechanically working the mixture to reduce the size of the complex agglomerates to less than about 100 microns. The softener helps protect the complex from the water.

The term "fabric conditioning (softening) agent" as used herein includes cationic and nonionic fabric softeners used alone and also in combination with each other. A preferred fabric softening agent of the present invention is a mixture of cationic and nonionic fabric softeners.

### (1) Fabric Conditioning (Softening) Agents

Examples of fabric softening agents that are especially useful in the substrate articles are the compositions described in US 5,348,667 and the references cited therein.

As stated hereinbefore, fabric conditioning agents can be nonionic, cationic, or mixtures thereof. These fabric conditioning agents and the compositions herein can be used for other purposes than fabric treating. E.g., the agents can be used to treat other substrates and/or for other end uses depending upon, e.g., the actives in the complex.

Examples of nonionic fabric softeners are fatty alcohols, fatty acids, fatty acid esters of, e.g., hydroxy, including polyhydroxy alcohols, including glycerine, sugars, etc., and/or fatty alcohol esters of carboxylic acids. More specific examples include sorbitan esters, C₁₂-C₂₆ - fatty alcohols, and fatty amines.

More biodegradable fabric softener compounds can be desirable. Biodegradability can be increased, e.g., by incorporating easily destroyed linkages into hydrophobic groups. Such linkages include ester linkages, amide linkages, and linkages containing unsaturation and/or hydroxy groups. Examples of such fabric softeners can be found in US 5,348,667 and the references cited therein.

A preferred fabric softener sheets of the present invention includes from about 30% to about 99%, preferably from about 40% to about 90%, of fabric conditioning (softening) agent. Preferably, said fabric softening agent is selected from cationic and nonionic fabric softeners and mixtures thereof. Preferably, said fabric softening agent comprises a mixture of about 5% to about 80% of a cationic fabric softener and about 10% to about 85% of a nonionic fabric softener by weight of said fabric treatment composition. The selection of the components is such that the resulting fabric treatment composition has a melting point above about 38°C and is flowable at dryer operating temperatures.

### (2) Dispensing Means

In a preferred substrate article embodiment, the fabric treatment compositions are provided as an article of manufacture in combination with a dispensing means such as a flexible substrate which effectively releases the composition in an automatic laundry (clothes) dryer. Such dispensing means can be designed for single usage or for multiple uses. The dispensing means can also be a "carrier material" that releases the fabric softener composition and then is dispersed and/or exhausted from the dryer.

The dispensing means will normally carry an effective amount of fabric treatment composition. Such effective amount typically provides sufficient fabric conditioning agent and/or anionic polymeric soil release agent for at least one treatment of a minimum load in an automatic laundry dryer. Amounts of fabric treatment composition for multiple uses, e.g., up to about 30, can be used. Typical amounts for a single article can vary from about 0.25 g to about 100 g, preferably from about 0.5 g to about 10 g, most preferably from about 0.8 g to about 5 g.

One such article comprises a sponge material releasably enclosing enough fabric treatment composition to effectively impart fabric soil release and softness benefits during several cycles of clothes. This multi-use article can be made by filling a hollow sponge with about 20 grams of the fabric treatment composition.

Highly preferred paper, woven or nonwoven "absorbent" substrates useful herein are fully disclosed in US 3,686,025. It is known that most substances are able to absorb a liquid substance to some degree; however, the term "absorbent" as used herein, is intended to mean a substance with an absorbent capacity (i.e., a parameter representing a substrate's ability to take up and retain a liquid) from 4 to 12, preferably 5 to 7, times its weight of water.

In another preferred embodiment flavored consumer products comprise one or more inventive flavor oil containing particles and are preferably products which are suitable for consumption. A product which is suitable for consumption is a product which is intended to be introduced into the human oral cavity, to remain there for a certain time and then either be swallowed, i.e. consumed (e.g. foodstuff, beverage) or removed from the oral cavity again (e.g. chewing gums and oral care compositions). This also includes substances or products which are intended to be taken in by humans or animals in the processed, partly processed or non-processed state. This furthermore includes all substances which are added to the foodstuff during its preparation, processing or working.

In a preferred embodiment inventive flavored products which are suitable for consumption are preferably selected from the group consisting of: powder products (preferably powdered beverage drink mixes, such as soft drink mixes, instant desserts in powder form, such as pudding powders), baked goods (preferably biscuits, cakes, muffins, waffles, baking mixtures), confectionery (preferably hard caramels, soft caramels, compressed products), dairy products (preferably yogurts, puddings, ice-creams), chocolate goods (preferably white, milk or plain chocolate, chocolate bars), fat compositions (preferably fillings for baked goods, such as e.g. biscuit fillings, fatty fillings for chocolate, fatty fillings for bars), chewing gums and sweets for chewing (preferably sugar-free, sugar-containing), cereals (preferably rolled oats, cornflakes), muesli mixtures (preferably conventional bulk muesli, students' mix, muesli bars, snacks and snack mixtures (preferably sweet popcorn, mixture of fruit pieces, nuts, nut bars, fruit-and-nut bars) and sprinkling mixtures (preferably toppings).

In another preferred embodiment the inventive flavored consumer products are selected from the group of dental care products, preferably dental creams, dental powders, tooth paste, tooth-cleaning foams, sugar-free candies for sucking, oral sprays, dental floss or (sugar-free) chewing gums.

Depending on the product type, the content of the inventive fragrance and/or flavor oil containing particles in an inventive consumer product is usually in the range of 0.05 - 10 wt.%, preferably in the range of 0.1 - 5 wt.%, based on the total weight of the finished perfumed and/or flavored consumer product. In a further preferred embodiment conventional base and/or auxiliary substances are additionally comprised in the inventive perfumed and/or flavored consumer products.

The preferred embodiments of the inventive consumer products can be combined in any possible way with each other and/or with the further preferred embodiments of the further subject matter of the present invention.

### EXAMPLES

The present invention is explained further with the aid of the following non-limiting examples, illustrating the parameters of and compositions employed within the present invention. Unless stated otherwise, all data, in particular percentages, parts and ratios are by weight unless otherwise indicated.

Abbreviations used: DPG: dipropylene glycol, EO = ethoxylated (e.g. 11 EO = 11 times ethoxylated); IPM: isopropyl myristate; PPG: polypropylene glycol.

### Materials / Instruments:

Monosodium phosphate (MSP) was obtained from Wego Chemical.

Capsul and Hi-Cap 100 were obtained from National Starch.

For determination of the average particle size a Beckman Coulter LS Particle Size Analyzer was used.

The following fragrance oils "A", "B" and "C" were used.

**Fragrance oil "A": a fruity fragrance, particularly suitable for use in detergent powder or fabric softener sheets**

| Material | Fragrance oil "A" |
|---|---|
| | parts by weight |
| AGRUMEX HC (2-tert.-butylcyclohexyl acetate) | 20.00 |
| ALDEHYDE C14 SO-CALLED | 9.00 |
| AMBRETTOLIDE | 0.10 |
| APHERMATE (alpha,3,3-trimethylcyclohexane-1-methanol formate) | 5.00 |
| ETHYLENE BRASSYLATE | 20.00 |
| HEXENOL CIS-3 | 0.10 |
| HEXENYL ACETATE CIS-3 | 0.20 |
| HEXYL CINNAMIC ALDEHYDE ALPHA | 2.50 |
| ISOAMYL BUTYRATE | 1.10 |
| ISOPROPYL MYRISTATE (IPM) | 26.20 |
| LIGUSTRAL | 0.50 |
| MACROLIDE® (15-pentadecanolide), 10% in IPM | 12.00 |
| 1 : 1 mixture of cis- and trans-3-methyl-γ-decalactone | 0.30 |
| PRENYL ACETATE | 3.00 |
| TOTAL: | 100.00 |

**Fragrance oil "B": a fruity floral fragrance, particularly suitable for use in antiperspirant**

| Material | Fragrance oil "B" |
|---|---|
| | parts by weight |
| ALDEHYDE C12 LAURIC, 10% in benzyl benzoate | 20.00 |
| ALDEHYDE C14 SO-CALLED | 15.00 |
| ALLYL AMYL GLYCOLATE | 40.00 |
| BENZYL ACETATE | 60.00 |
| CITRONELLOL | 60.00 |
| CYCLABUTE | 10.00 |
| DIHYDROMYRCENOL | 100.00 |
| ETHYL METHYL BUTYRATE-2 | 8.00 |
| EVERNYL | 10.00 |
| FARENAL® | 8.00 |
| GERANYL ACETATE | 3.00 |
| GLOBALIDE® | 100.00 |
| MUGETANOL (1-(4-isopropylcyclohexyl)ethanol) | 150.00 |
| HERBAFLORAT | 30.00 |
| HERBYL PROPIONATE | 50.00 |
| HEXENOL CIS-3 | 3.00 |
| HEXENYL ACETATE CIS-3 | 10.00 |
| IONONE BETA | 80.00 |
| ISO E SUPER® | 201.80 |
| ISOLONGIFOLANONE | 0.10 |
| KEPHALIS | 0.10 |
| LEMON OIL TERPENES FLAVOR NATURAL | 10.00 |
| LIGUSTRAL | 6.00 |
| LINALOOL | 80.00 |
| MANZANATE (ethyl 2-methylpentanoate) | 2.00 |
| ORANGE OIL BRASIL | 23.00 |
| PRENYL ACETATE | 5.00 |
| SANDRANOL® | 10.00 |
| VANILLIN, 10% in DPG | 5.00 |
| TOTAL: | 1,100.00 |

**Fragrance oil "C": a powdery fragrance, particularly suitable for use in talc preparations**

| Material | Fragrance oil "C" |
|---|---|
| | parts by weight |
| AGRUNITRIL | 10.00 |
| ALDEHYDE C10 | 40.00 |
| ALDEHYDE C12 MNA | 2.00 |
| ALLYL AMYL GLYCOLATE | 30.00 |
| ALLYL CYCLOHEXYL PROPIONATE | 100.00 |
| AMBROCENIDE®, 10% in DPG | 3.00 |
| AMBROX DL | 1.00 |
| ANETHOL | 40.00 |
| BOURGEONAL | 5.00 |
| CALONE 1951 | 1.00 |
| CAMPHOR | 25.00 |
| CEDAR LEAF OIL | 25.00 |
| CEDRAMBER | 100.00 |
| CITRAL | 2.00 |
| COUMARIN | 3.00 |
| CYCLOGALBANAT® | 20.00 |
| DAMASCONE DELTA | 1.00 |
| DIHYDRO MYRCENOL | 700.00 |
| DODECENAL TRANS-2 | 1.00 |
| EUCALYPTUS OIL GLOBULUS | 25.00 |
| EVERNYL | 10.00 |
| FARENAL® | 50.00 |
| FLORAZON | 70.00 |
| GERANIUM OIL | 3.00 |
| GLOBALIDE® | 30.00 |
| GLOBANONE® | 30.00 |
| HEDIONE® | 80.00 |
| HEXENOL CIS-3 | 5.00 |
| HEXENYL ACETATE CIS-3 | 15.00 |
| HEXYL SALICYLATE | 50.00 |
| ISO E SUPER® | 100.00 |
| ISOBUTYL QUINOLINE | 1.00 |
| ISOEUGENOL | 1.00 |
| LIGUSTRAL | 5.00 |
| LINALOOL | 103.00 |
| MELONAL® | 20.00 |
| PATCHOULI OIL | 1.00 |
| SANDRANOL® | 46.00 |
| STYRALYL ACETATE | 17.00 |
| THYMOL, 10% in DPG | 4.00 |
| TIMBEROL® | 2.00 |
| VERNALDEHYDE (1-methyl-4-isohexyl-hexahydrobenzaldehyde) | 3.00 |
| VERTOFIX | 20.00 |
| TOTAL: | 1,800.00 |

The following flavor oils "D" and "E" were used.

Flavor oil "D": a 1 : 1 mixture of natural lemon and grapefruit essentail oils.

**Flavor oil "E": (synthetic peppermint oil)**

| Material | Flavor oil "E" |
|---|---|
| | parts by weight |
| Isobutyraldehyde | 0.5 |
| 3-Octanol | 0.5 |
| Dimethyl sulfide | 0.5 |
| trans-2-Hexenal | 1.0 |
| cis-3-Hexenol | 1.0 |
| 4-Terpineol, natural | 1.0 |
| Isopulegol | 1.0 |
| Piperitone, natural, from eucalyptus | 2.0 |
| Linalool | 3.0 |
| 8-Ocimenyl acetate, 10 wt.% in triacetin | 5.0 |
| Isoamyl alcohol | 10.0 |
| Isovaleraldehyde | 10.0 |
| alpha-Pinene | 25.0 |
| beta-Pinene, natural | 25.0 |
| Neomenthol, racemic | 40.0 |
| Eucalyptol (1,8-cineol), natural | 50.0 |
| L-Menthyl acetate | 70.0 |
| L-Menthone | 220.0 |
| D-Isomenthone | 50.0 |
| L-Menthol | 484.5 |
| Total: | 1,000.00 |

### Example 1 (all spray-dried particles)

The Process of spray-drying was performed at the following conditions:
A spray dryer such as Anhydro #1 lab dryer equipped with a rotary atomizer or two fluid nozzle or high pressure nozzle.

Drying conditions were: inlet at 190 °C and outlet at 90 °C, each plus or minus 5 °C. Process equipment can encompass any spray-dryer that can emulate these conditions.

### Example 1a:

| Mixture before drying | Results after spray-drying |
|---|---|
| Fragrance Oil "A" = 42% | Total Fragrance Oil "A" = 65.43% |
| Capsul starch = 9.36% | Surface Fragrance Oil = 2.32% |
| MSP = 2.34 % | Residual Water = 0.83% |
| Mannitol = 2.34% | |
| Water at 40 °C = 40% | |

| | |
|---|---|
| % = to wt.-% based on the mixture before drying or the results after spray-drying. | |

### Example 1b (not according to the invention):

| Mixture before drying | Results after spray-drying |
|---|---|
| Fragrance Oil "B" = 30% | Total Fragrance Oil "B" = 59.95% |
| Hi-Cap 100 starch = 10% | Surface Fragrance Oil = 0.57% |
| MSP = 10% | Residual Water = 1.28% |
| Water at 40 °C = 50% | |

| | |
|---|---|
| % = to wt.-% based on the mixture before drying or the results after spray-drying. | |

### Example 1c:

| Mixture before drying | Results after spray-drying |
|---|---|
| Fragrance Oil "B" = 42% | Total Fragrance Oil "B" = 68.79% |
| Hi-Cap 100 starch = 11.7% | Surface Fragrance Oil = 1.42% |
| MSP = 6.3% | Residual Water = 0.97% |
| Water at 40 °C = 40% | |

| | |
|---|---|
| % = to wt.-% based on the mixture before drying or the results after spray-drying. | |

The average particle size was 19.2 microns.

### Example 1d:

| Mixture before drying | Results after spray-drying |
|---|---|
| Fragrance Oil "C" = 45% | Total Fragrance Oil "C" = 74.06% |
| Hi-Cap 100 starch = 9.75% | Surface Fragrance Oil = 2.95% |
| MSP = 5.25% | Residual Water = 1.98% |
| Water at 40 °C = 40% | |

| | |
|---|---|
| % = to wt.-% based on the mixture before drying or the results after spray-drying. | |

### Example 1e:

| Mixture before drying | Results after spray-drying |
|---|---|
| Flavor Oil "D" = 43% | Total Flavor Oil "D" = 68.1% |
| Hi-Cap 100 starch = 9.75% | Surface Flavor Oil = 2.3% |
| MSP = 5.25% | Residual Water = 1.28% |
| Water at 40 °C = 40% | |

| | |
|---|---|
| % = to wt.-% based on the mixture before drying or the results after spray-drying. | |

### Example 1f:

| Mixture before drying | Results after spray-drying |
|---|---|
| Flavor Oil "E" = 41% | Total Flavor Oil "E" = 66.92% |
| Hi-Cap 100 starch = 11.5% | Surface Flavor Oil = 1.52% |
| MSP = 6.4% | Residual Water = 0.91% |
| Water at 40 °C = 40% | |

| | |
|---|---|
| % = to wt.-% based on the mixture before drying or the results after spray-drying. | |

### Example 1g:

| Mixture before drying | Results after spray-drying |
|---|---|
| Fragrance Oil "A" = 47.3% | Total Fragrance Oil "A" = 76.26% |
| Hi-Cap 100 starch = 7.8% | Surface Fragrance Oil = 3.97% |
| MSP = 4.7 % | Residual Water = 0.31% |
| Water at 40 °C = 40% | |

| | |
|---|---|
| % = to wt.-% based on the mixture before drying or the results after spray-drying. | |

### Example 1-P1: Free perfume oil

| Components | Perfume oil P1 |
|---|---|
| | parts by weight |
| 10-undecenal, 10% in DPG | 2.00 |
| Vertocitral (2,4-dimethyl-3-cyclohexencarboxaldehyde) | 0.50 |
| cis-3-Hexenyl acetate, 10% in DPG | 7.50 |
| Allylamylglycolate | 2.00 |
| Melonal (2,6-dimethyl-5-hepten-1-al) | 0.50 |
| Bergamot oil | 70.00 |
| Dihydromyrcenol | 80.00 |
| Cyclogalbanate (allylcyclohexyloxy acetate) | 20.00 |
| Terpinyl acetate | 40.00 |
| Litsea cubeba oil | 2.00 |
| Lemon oil | 50.00 |
| Orange oil | 20.00 |
| Grapefruit oil | 10.00 |
| Lavandin oil abrialis | 10.00 |
| Isobornyl acetate | 3.00 |
| Lilial (2-methyl-3-(4-tert-butyl-phenyl)propanal) | 10.00 |
| Calone® 1951 (7-methyl-2*H*-1,5-benzodioxepin-3(4*H*)-one) | 2.50 |
| Florhydral® (3-(3-isopropylphenyl)butanal) | 1.50 |
| Florol® (2-isobutyl-4-methyltetrahydro-2*H*-pyran-4-ol) | 12.00 |
| Tetrahydrolinalool | 75.00 |
| Geranium oil | 5.00 |
| Isodamascon (1-(2,4,4-trimethyl-2-cyclohexen-1-yl)-2-buten-1-one), 10% in DPG | 2.00 |
| Resedafol, 10% in DPG [2-(1-propoxyethoxy)ethyl]benzol | 1.00 |
| Methyl dihydrojasmonate (Hedione® HC) | 158.00 |
| L-menthylmethylether | 50.00 |
| Jessemal (3-butyl-5-methyl tetrahydropyran-4-yl-acetate) | 4.00 |
| Benzyl salicylate | 10.00 |
| Anethol | 3.00 |
| Methylcedrylketone | 50.00 |
| Iso E Super® ⁺⁺ | 25.00 |
| Ambrocenide® ((4aR,5R,7aS,9R)-octahydro-2,2,5,8,8,9a-hexamethyl-4H-4a,9-methanoazuleno(5,6-d)-1,3-dioxol), 1% in DPG | 3.00 |
| Timberol (1-(2,2,6-trimethylcyclohexyl)hexan-3-ol) | 2.00 |
| Patchouli oil | 3.50 |
| Evernyl® (methyl-2,4-dihydroxy-3,6-dimethylbenzoate) | 1.50 |
| Labdanum absolute, 20% in DPG | 2.00 |
| Amber Core (1-[[2-(1,1-dimethylethyl)cyclohexyl]oxy]-2-butanol) | 10.00 |
| Ambraketal (dodecahydro-3,8,8,11a-tetramethyl-5*H*-3,5a-epoxynaphth[2,1-c]oxepin) | 1.50 |
| Hydroxyambran®(2-cyclododecylpropanol), 50% in DPG | 5.00 |
| Macrolide® (15-cyclopentadecanolide) | 35.00 |
| Globalide® (15-pentadec-(11/12)-enolide) | 20.00 |
| Globanone® ((E/Z)-8-cyclohexadecenone) | 20.00 |
| Isopropylmyristate | 170.00 |
| TOTAL: | 1,000.00 |

| | |
|---|---|
| ++: Octahydro-2,3,8,8-tetramethyl-2-acetonaphthone and 2-acetyl-1,2,3,4,6,7,8-octahydro-2,3,8,8-tetramethylnaphthaline | |

### Example 1-P2: Free perfume oil with rose odor

| Components | Perfume oil P2 |
|---|---|
| | parts by weight |
| Acetophenone, 10% in DPG | 10.00 |
| n-Undecanal | 5.00 |
| Aldehyde C14 so-called (peach aldehyde) | 15.00 |
| Allylamylglycolate, 10% in DPG | 20.00 |
| Amylsalicylate | 25.00 |
| Benzyl acetate | 60.00 |
| Citronellol | 80.00 |
| d-Limonen | 50.00 |
| Trans-9 decenol | 15.00 |
| Dihydromyrcenol | 50.00 |
| Dimethylbenzyl carbinyl acetate | 30.00 |
| Diphenyl oxide | 5.00 |
| Eucalyptus oil | 10.00 |
| Geraniol | 40.00 |
| Nerol | 20.00 |
| Geranium oil | 15.00 |
| Hexenol cis-3, 10% in DPG | 5.00 |
| Hexenyl salicylate cis-3 | 20.00 |
| Indol, 10% in DPG | 10.00 |
| Alpha-lonone | 15.00 |
| Beta-lonone | 5.00 |
| Lilial (2-methyl-3-(4-tert-butyl-phenyl)propanal) | 60.00 |
| Linalool | 40.00 |
| Methylphenyl acetate | 10.00 |
| Phenylethyl alcohol | 275.00 |
| Styrene acetate | 20.00 |
| Terpineol | 30.00 |
| Tetrahydrolinalool | 50.00 |
| Cinnamon alcohol | 10.00 |
| | |
| TOTAL: | 1,000.00 |

### Example 1-P3: Free perfume oil with woody musk odor

| Components | Perfume oil P3 |
|---|---|
| | Weight % |
| Ambrettolide | 5.00 |
| p-tert-Butyl cyclohexyl acetate | 10.00 |
| Cedrol | 10.00 |
| Exaltolide | 5.00 |
| Galaxolide, 50% in IPM | 15.00 |
| Hexadecanolide | 1.00 |
| Gamma-n-methyl ionone | 10.00 |
| Iso E Super | 8.00 |
| Musk indanone | 7.00 |
| Musk tibetine | 5.00 |
| Patchouli alcohol | 7.00 |
| Vetiveryl acetate | 5.00 |
| Methyl dihydrojasmonate | 8.00 |
| Coumarin | 3.00 |

### Example 2: Talc powder

| % Wt | Ingredient | Supplier |
|---|---|---|
| 98.50 | Ultra Talc 3000 | Ultra Products |
| 0.50 | Free perfume oil | Symrise |
| 1.00 | Particles of Example 1d | Example 1d |

Compounding Procedure: mix talc thoroughly with free perfume oil and Particles according to the present invention.

### Example 3: Antiperspirant stick

| Part | % Wt | Ingredient | Supplier |
|---|---|---|---|
| 1 | 2.00 | Gelling Agent GP-1 (Dibutyl Lauroyl Glutamide) | Ajinonoto |
| 2 | 6.00 | Casid HSA (Hydroxystearic Acid) | CasChem |
| 3 | 48.05 | DC 244 Fluid (Cyclomethicone) | Dow Corning |
| 4 | 13.9 | Eutanol G (Octyldodecanol) | Cognis Care |
| 5 | 1.25 | Performathox 450 (C20-40 Pareth-10) | New Phase Tech-nologies |
| 6 | 1.25 | Performathox 480 (C20-40 Pareth 40) | New Phase Technologies |
| 7 | 0.50 | Performacol 425 (C20-40 Alcohols) | New Phase Technologies |
| 8 | 0.05 | Versene NA (Disodium EDTA) | Dow Chemical |
| 9 | 25.0 | Reach AZP-908 SUF (Aluminum Zirconium Tetrachlorohydrex Gly) | Reheis |
| 10 | 0.75 | Free perfume oil 1-P1 | Example 1-P1 |
| 11 | 1.25 | Particles of Example 1b (not according to the invention) | Example 1b |

Compounding Procedure: add the ingredient parts 1 - 7 to a suitable vessel and heat to 80 °C. Mix until all waxes are melted and clear homogeneous solution is achieved. Allow to cool to 70 °C with continued mixing. Add ingredients 8 and 9 with mixing until fully dispersed. Continue to mix while cooling to 65 °C. Add ingredients 10 and 11, and mix and pour into stick containers.

### Example 4: Antiperspirant aerosol

| Part | % Wt | Ingredient | Supplier |
|---|---|---|---|
| 1 | 10.00 | DC Fluid 244 (Cyclomethicone) | Dow Corning |
| 2 | 7.50 | Micro Dry (Aluminum Chlorohydrate) | Reheis |
| 3 | 1.25 | Myritol PC (Propylene Glycol Dicaprylate/Dicaprate) | Cognis Care |
| 4 | 0.75 | Free perfume oil of Example 1-P2 | Example 1-P2 |
| 5 | 0.50 | Particles of Example 1c (average particle size: 32 microns) | Example 1c |
| 6 | 80.0 | Hydrocarbon Propellant A-46 | |

Compounding Procedure: combine parts 1 - 5 in a suitable vessel. Mix until a homogeneous dispersion is achieved. Fill into aerosol cans, crimp on valve and pressure fill propellant (part 6) (a 20/80 blend of propane/isobutane).

### Example 5: Fabric Softener Sheet

| Part | % Wt | Ingredient | Supplier |
|---|---|---|---|
| 1 | 97.00 | Varisoft DS-150 | Degussa |
| 2 | 1.50 | Free perfume oil | Symrise |
| 3 | 1.50 | Particles of Example 1a | Example 1a |

Compounding Procedure: Melt Quat (part 1), add Free perfume oil and Particles of Example 1a. Using a spatula spread 1.1 g of the mixture onto a 6 x 9 inches non-woven sheet.

### Example 6: Non-phosphate powdered detergent

| % Wt | Ingredient | Supplier |
|---|---|---|
| 6.00 | Tomadol 25-7 (Peg-7 Pareth 12-15 | Tomah Products |
| 2.00 | Tomadol 25-3 (Peg-3 Pareth 12-15) | Tomah Products |
| 57.55 | Sodium metasilicate, pentahydrate | |
| 33.00 | Sodium Carbonate | |
| 1.00 | Carboxymethylcellulose | |
| 0.15 | Free perfume oil of Example 1-P3 | Symrise |
| 0.30 | Particles of Example 1g | Example 1g |

### Example 7: Antiperspirant stick

| Ingredient | % by weight | % by weight |
|---|---|---|
| Phenyl Trimethicon (SilCare TM Silicone 15 M 50) | 13.50 | 13.50 |
| Cetearyl alcohol | To 100 | To 100 |
| Cetiol CC (dicaprylyl carbonate) | 13.50 | 13.50 |
| Stearic acid | 3.50 | 3.50 |
| PEG-40-hydrated castor oil (Emulsogen TM HCO 040) | 4.10 | 4.10 |
| PEG-8 distearate (Cithrol 4 DS) | 4.10 | 4.10 |
| Petrolatum | 6.90 | 6.90 |
| Aluminum hydrochlorate | 13.80 | 13.80 |
| Aluminum zirconium trichlorohydrex gly | 20.00 | 19.50 |
| Neo Heliopan® Hydro (phenylbenzimidazole sul-phonic acid, Symrise) | 2.00 | 0.50 |
| 2,2-dimethyl-3-phenylpropanol | - | 0.25 |
| Ethylhexylglycerine (octoxyglycerin) | - | 0.30 |
| 1,1-dimethyl-3-phenylpropanol | - | 0.25 |
| Particles according to Example 1c | 0.50 | - |
| Perfume oil according to Example 1-P1 | 0.55 | - |
| Particles according to Example 1d | - | 0.30 |
| Perfume oil according to Example 1-P2 | - | 1.00 |

### Example 8: Suspension roll-on

| Ingredient | % by weight | % by weight |
|---|---|---|
| Silicone | To 100 | To 100 |
| Ethylhexylglycerine (octoxyglycerin) | 1.00 | 1.00 |
| Quaternium-18 hectorite | 13.00 | 13.20 |
| Aluminum hydrochlorate, powder | 21.00 | 20.00 |
| 1,1-dimethyl-3-phenylpropanol | 0.25 | 0.50 |
| 4-methyl-4-phenyl-2-pentanol | 0.10 | - |
| Particles according to Example 1c | 0.25 | - |
| Perfume oil according to Example 1-P2 | 1.00 | - |
| Particles according to Example 1b (not according to the invention) | - | 0.25 |
| Perfume oil according to Example 1-P1 | - | 0.85 |

### Example 9: Suspension stick

| Ingredient | % by weight | % by weight |
|---|---|---|
| Stearyl alcohol | 20.00 | 20.00 |
| Cyclomethicone | To 100 | To 100 |
| PPG-14 butyl ether | 2.00 | 2.00 |
| Hydrated castor oil | 1.00 | 1.00 |
| Talc | 2.00 | 2.00 |
| Aluminum hydrochlorate, powder | 20.00 | 20.00 |
| Triclosan® (5-chloro-2-(2,4-dichlorphenoxy)phenol) | 0.30 | - |
| Ethylhexylglycerine (octoxyglycerin) | 0.50 | 0.80 |
| 1,1-dimethyl-3-phenylpropanol | 0.30 | 0.40 |
| 2,2-dimethyl-3-phenylpropanol | 0.30 | 0.15 |
| Anis alcohol | 0.10 | - |
| Particles according to Example 1b (not according to the invention) | 0.50 | - |
| Perfume oil according to Example 1-P1 | 0.55 | - |
| Particles according to Example 1g | - | 0.30 |
| Perfume oil according to Example 1-P2 | - | 0.80 |

### Example 10: Heavy duty granular detergent

| Ingredient | % by weight | % by weight |
|---|---|---|
| C₁₂ Linear alkylbenzene sulfonate (Na) | 9.00 | 9.00 |
| C₁₄₋₁₅ Alkyl ethoxy /EO = 0.6) sulfate (Na) | 1.60 | 1.60 |
| C₁₂₋₁₈ Alkyl sulfate | 5.70 | 5.70 |
| Polyacrylate (MW = 4500) | 3.20 | 3.20 |
| Aluminosilicate | 26.30 | 26.30 |
| Sodium silicate | 0.60 | 0.60 |
| Sodium carbonate | 27.90 | 27.90 |
| Sodium sulfate | 8.90 | 8.90 |
| Optical Brightener | 0.20 | 0.20 |
| Perborate | 1.00 | 1.00 |
| Cellulase (5 CEVU/g) CAREZYME® | 0.60 | 0.60 |
| Protease (0.0062 AU/g) according to US 5,185,258 | 0.30 | 0.30 |
| Lipase (206 LUI/g) LIPOLASE® | 0.20 | 0.20 |
| Nonionic | 3.00 | 3.00 |
| Particles according to Example 1g | 0.25 | - |
| Perfume oil according to Example 1-P3 | 0.30 | - |
| Particles according to Example 1a | - | 0.20 |
| Perfume oil according to Example 1-P3 | - | 0.35 |
| Miscellaneous (water and other minors) | To 100 | To 100 |

### Example 11: Heavy duty liquid detergent

| Ingredient | % by weight | % by weight |
|---|---|---|
| C₁₄₋₁₅ Alkyl polyethoxylate (2.25) sulfonic acid | 23.00 | 12.50 |
| C₁₂₋₁₃ Linear alkyl benzene sulfonic acid | - | 11.46 |
| 1,2 Propanediol | 10.50 | 10.50 |
| Monoethanolamine | 12.50 | 12.50 |
| C₁₂₋₁₃ Alkyl polyethoxylate (6.5) | 6.00 | 6.00 |
| Ethanol | 3.80 | 3.80 |
| Polyhydroxy C₁₂₋₁₄ fatty acid amide | 9.00 | 9.00 |
| C₁₂₋₁₄ Coconut fatty acid | 9.00 | 9.00 |
| Citric acid | 6.00 | 6.00 |
| DTPA (Diethylenetriaminepentaethylene phosphonic acid) | 0.95 | 0.95 |
| Sodium formate | 0.14 | 0.14 |
| Boric acid | 2.40 | 2.40 |
| Tetraethylenepentaamine ethoxylate (15-18) | 1.00 | 1.00 |
| Soil release polymer | 0.46 | 0.46 |
| Enzymes (protease, lipase, cellulase) | 2.55 | 2.55 |
| Silicone antifoam composition | 0.04 | 0.04 |
| Poly(4-vinylpyridine)-N-oxide (PVNO) | 0.10 | 0.10 |
| Brightener - Tinopal UNPA-GX | 0.20 | 0.20 |
| Particles according to Example 1a | 0.15 | - |
| Perfume oil according to Example 1-P2 | 0.10 | - |
| Particles according to Example 1c | - | 0.10 |
| Perfume oil according to Example 1-P3 | - | 0.15 |
| Miscellaneous (water and other minors) | To 100 | To 100 |

### Example 12: Compact granular detergent compositions

| Ingredient | % by weight | % by weight |
|---|---|---|
| C₁₁₋₁₄ Linear alkylbenzene sulfonate (Na) | 11.40 | - |
| C₁₂₋₁₄ N-methyl glucamide | - | 13.00 |
| Tallow alkyl sulfate | 1.80 | 1.80 |
| C₄₅ Alkyl sulfate | 3.00 | 3.00 |
| C₄₅ Alcohol (7 EO) | 4.00 | 4.00 |
| Tallow alcohol (11 EO) | 1.80 | 1.80 |
| Dispersant | 0.07 | 0.07 |
| Silicone fluid | 0.80 | 0.80 |
| Trisodium citrate | 14.00 | 14.00 |
| Citric acid | 3.00 | 3.00 |
| Zeolites (incl. Zeolite A and Zeolite X) | 32.50 | 32.50 |
| Maleic acid - acrylic acid copolymer | 5.00 | 5.00 |
| Cellulase (active protein) | 0.04 | 0.04 |
| Alkalase | 0.60 | 0.60 |
| Lipase | 0.36 | 0.36 |
| Sodium silicate | 2.00 | 2.00 |
| Sodium sulfate | 3.50 | 3.50 |
| Poly(4-vinylpyridine)-N-oxide (PVNO) | 0.10 | - |
| N-vinylpyrrolidone/N-vinylimidazole copolymer - MW 10,000 (PVPVI) | - | 0.20 |
| Brightener - Tinopal UNPA-GX | 0.20 | - |
| Brightener - Tinopal 5BM-GX | - | 0.20 |
| Particles according to Example 1a | 0.25 | - |
| Perfume oil according to Example 1-P3 | 0.30 | - |
| Particles according to Example 1c | - | 0.25 |
| Perfume oil according to Example 1-P1 | - | 0.25 |
| Miscellaneous (water and other minors) | To 100 | To 100 |

### Example 13: Sugar-reduced instant beverage mix

A dry mixture comprising the ingredients of the following table was prepared:

| | |
|---|---|
| sugar (sucrose) | 82.169 % |
| citric acid | 11.58 % |
| trisodium citrate | 0.70 % |
| tricalcium phosphate | 0.60 % |
| ascorbic acid (vitamin C) | 0.66 % |
| Grindsted JU 543 stabilizer system | 0.90 % |
| Na-saccharin | 0.561 % |
| spray-dried orange flavor, including yellow colorant tartrazine | 1.33 % |
| Particles of Example 1e | 1.50 % |

| | |
|---|---|
| % refers to wt.-% based on the dry mixture. | |

### Example 14: Chewing gum with particles according to the invention

| | |
|---|---|
| sorbitol | 40.0 % |
| gum base | 32.0 % |
| calcium carbonate | 15.0 % |
| glycerin | 1.5 % |
| mannitol | 4.5 % |
| sorbitol liquid | 3.0 % |
| liquid peppermint flavor | 0.5 % |
| encapsulated menthol | 1.0 % |
| encapsulated aspartame | 0.5 % |
| particles of Example 1f | 1.0 % |

| | |
|---|---|
| % refers to wt.-% based on the chewing gum. | |

### Example 15: Chewing gum with particles according to the invention

Chewing gum base K2 comprised 28.5 % terpene resin, 33.9 % polyvinyl acetate (MW = 14,000), 16.25 % hydrogenated plant oil, 5.5 % mono- and diglycerides, 0.5 % polyisobutene (MW 75,000), 2.0 % butyl rubber (isobutene/isoprene copolymer), 4.6 % amorphous silicon dioxide (water content approx. 2.5 %), 0.05 % antioxidant tert-butylhydroxytoluene (BHT), 0.2 % lecithin, and 8.5% calcium carbonate. Chewing gum base K2 and the chewing gums can be prepared analogously to US 6,986,907, incorporated herein in its entirety.

| | I (wt.%) | II (wt.%) |
|---|---|---|
| Chewing gum base K2 | 25.30 | 26.30 |
| Sorbitol | Ad 100 | Ad 100 |
| Glycerol | 2.40 | 2.40 |
| Lecithin | 7.00 | 7.00 |
| Aspartame | 0.14 | 0.14 |
| Encapsulated aspartame | 0.68 | 0.68 |
| Menthol, spray-dried | 0.25 | 0.50 |
| Lemon aroma, spray-dried | - | - |
| Particles of Example 1f | 1.25 | 0.95 |

| | | |
|---|---|---|
| The chewing gums of recipe (I) were shaped as strips, and those of recipe (II) were shaped as pellets. | | |

### Example 16: Non-stick chewing gum with particles according to the invention

Chewing gum base K1 comprised 2.0 % butyl rubber (isobutene/isoprene copolymer, MW 400,000), 6.0 % polyisobutene (MW = 43,800), 43.5 % polyvinyl acetate (MW 12,000), 31.5 % polyvinyl acetate (MW = 47,000), 6.75 % triacetin and 10.25 % calcium carbonate. Chewing gum base K1 and the chewing gums (I) and (II) can be prepared analogously to US 5,601,858, incorporated herein in its entirety.

| | I (wt.%) | II (wt.%) |
|---|---|---|
| Chewing gum base K1 | 26.00 | 26.00 |
| Triacetin | 0.25 | 0.25 |
| Lecithin | 0.50 | 0.50 |
| Sorbitol, crystalline | Ad 100 | Ad 100 |
| Mannitol | 15.30 | 15.20 |
| Glycerol | 12.10 | 12.00 |
| Aspartame | 0.17 | 0.17 |
| Encapsulated aspartame | 1.08 | 1.08 |
| Amorphous silica | 1.00 | 1.00 |
| Cottonseed oil | 0.50 | 0.50 |
| Polyoxyethylene sorbitan monolaurate (E-432) | 1.00 | 1.00 |
| Menthone glycerine acetal (Frescolat^{®} MGA) | - | 0.15 |
| Encapsulated spearmint flavor (contains I-carvone) | 0.20 | 0.10 |
| Encapsulated wintergreen flavor (contains methyl salicylate) | - | 0.10 |
| Particles of Example 1f | 1.40 | 1.00 |

| | | |
|---|---|---|
| The chewing gums of recipe (I) were shaped as strips, and those of recipe (II) were shaped as pellets. | | |

Having thus described the invention in detail, it will be clear to those skilled in the art that various changes may be made without departing from the scope of the invention and the invention is not to be considered limited to what is described in the specification.

## Claims

1. A fragrance and/or flavor oil containing particle consisting of:
(a) 65 - 80 wt.% fragrance and/or flavor oil,
(b) 5 to 25 wt.% modified starch wherein the modified starch is octenylsuccinated starch,
(c) 5 to 25 wt.% of one or more phosphate salts selected from mono alkaline metal phosphates,
(d) 0 - 10 wt.% of one or more additional ingredients,
wherein the weight percent values are based on the total dry weight of the particle, and wherein the particles comprise no other modified starch.

2. The fragrance and/or flavor oil containing particle according to claim 1 consisting of:
(a) 65 - 78 wt.% fragrance and/or flavor oil, and/or
(b) 10 - 25 wt.% chemically modified starch wherein the modified starch is octenylsuccinated starch, and/or
(c) 5 - 15 wt.% of monosodium and/or monopotassium phosphate and/or
(d) 0 - 5 wt.% of one or more additional ingredients,
wherein the weight percent values are based on the total dry weight of the particle, and wherein the particles comprise no other modified starch.

3. The fragrance and/or flavor oil containing particle according to claim 2 consisting of:
(a) 65 - 75 wt.% fragrance or flavor oil,
(b) 15 - 22 wt.% of chemically modified starch wherein the modified starch is octenylsuccinated starch,
(c) 8 - 12 wt.% of monosodium phosphate and
(d) 0 - 5 wt.% additional ingredients
and wherein the particles comprise no other modified starch.

4. The fragrance and/or flavor oil containing particle according to any of claims 1 to 3 having:
(i) an particle size equal to or less than 300 microns, and/or
(ii) a residual water content equal to or less than 3 wt.%, and/or
(iii) an amount of surface oil of equal to or less than 4 wt.%,
wherein all weight percent values are based on the total dry weight of the particles.

5. The fragrance and/or flavor oil containing particle according to claim 4 having:
(i) an average particle size in the range of 5 to 125 microns, and/or
(ii) a residual water content in the range of 0.1 to 2.5 wt.%, and/or
(iii) an amount of surface oil of less than 3 wt.%,
wherein all weight percent values are based on the total dry weight of the particles.

6. A process for producing the fragrance and/or flavor oil containing particles according to any one of claims 1 to 5 consisting of:
i. forming a mixture consisting of the following constituents (a) through (e)
(a) 20 - 56 wt.% fragrance and/or flavor oil,
(b) 2 to 17.5 wt.% modified starch, wherein the modified starch is octenylsuccinated starch,
(c) 2 to 17.5 wt.% of one or more phosphate salts selected from mono alkaline metal phosphates,
(d) 0 - 7 wt.% of one or more additional ingredients and
(e) 30 - 60 wt%. of water,
in each case based on the total weight of the mixture and then
ii. drying the mixture of step i) to result in the fragrance and/or flavor oil containing particles wherein the particles comprise no other modified starch.

7. The process for producing the fragrance and/or flavor oil containing particles according to claim 6, wherein the particles comprise no other modified starch, wherein one or more of the following amounts of constituents (a) through (e) are provided in step i):
(a) 27.5 - 52 wt.% fragrance and/or flavor oil and/or
(b) 2.25 - 11.25 wt.% modified starch wherein the modified starch is octenylsuccinated starch, and/or
(c) 2.25 - 11.25 wt.% of one or more phosphate salts selected from mono alkaline metal phosphates, and/or
(d) 0 - 6.5 wt.% of one or more additional ingredients, and/or
(e) 35 - 55 wt.% of water,
in each case based on the total weight of the mixture

8. The process for producing the fragrance and/or flavor oil containing particles according to claim 6 or 7, wherein the drying step ii) is a spray-drying step.

9. A fragrance and/or flavor containing particle obtainable by the process according to any one of claims 6 to 8.

10. A perfumed and/or flavored consumer product comprising one or more fragrance and/or flavor oil containing particles according to any one of claims 1 to 5.

11. A method of perfuming or flavoring a consumer product by adding one or more fragrance and/or flavor oil containing particles according to any one of claims 1 to 5 to the consumer product.

12. Use of one or more of the fragrance and/or flavor oil containing particles according to any one of claims 1 to 5 to perfume and/or flavor a consumer product.

## Patentansprüche

1. Duft- und/oder Aromaöl-enthaltender Partikel bestehend aus:
(a) 65 - 80 Gew.-% Duft- und/oder Aromaöl,
(b) 5 bis 25 Gew.-% modifizierte Stärke, wobei die modifizierte Stärke octenyl-succinylierte Stärke ist,
(c) 5 bis 25 Gew.-% eines oder mehrerer Phosphatsalze ausgewählt aus monoalkalinen Metallphosphaten,
(d) 0 - 10 Gew.-% eines oder mehrerer zusätzlicher Inhaltsstoffe,
wobei die Gewichtsprozent-Angaben auf das Gesamttrockengewicht des Partikels bezogen sind und wobei die Partikel keine andere modifizierte Stärke enthalten.

2. Duft- und/oder Aromaöl-enthaltender Partikel nach Anspruch 1 bestehend aus:
(a) 65 - 78 Gew.-% Duft- und/oder Aromaöl, und/oder
(b) 10 - 25 Gew.-% chemisch-modifizierte Stärke, wobei die modifizierte Stärke octenyl-succinylierte Stärke ist, und/oder
(c) 5 - 15 Gew.-% Mononatrium- und/oder Monokaliumphosphat und/oder
(d) 0 - 5 Gew.-% eines oder mehrerer zusätzlicher Inhaltsstoffe,
wobei die Gewichtsprozent-Angaben auf das Gesamttrockengewicht des Partikels bezogen sind und wobei die Partikel keine andere modifizierte Stärke enthalten.

3. Duft- und/oder Aromaöl-enthaltender Partikel nach Anspruch 2 bestehend aus:
(a) 65 - 75 Gew.-% Duft- oder Aromaöl,
(b) 15 - 22 Gew.-% chemisch-modifizierte Stärke, wobei die modifizierte Stärke octenyl-succinylierte Stärke ist,
(c) 8 - 12 Gew.-% Mononatriumphosphat und
(d) 0 - 5 Gew.-% zusätzliche Inhaltsstoffe,
und wobei die Partikel keine andere modifizierte Stärke enthalten.

4. Duft- und/oder Aromaöl-enthaltender Partikel nach einem der Ansprüche 1 bis 3 mit:
(i) einer Partikelgröße gleich oder weniger als 300 Mikronen, und/oder
(ii) einem Rest-Wassergehalt gleich oder weniger als 3 Gew.-%, und/oder
(iii) einer Menge an Oberflächenöl von gleich oder weniger als 4 Gew.-%,
wobei alle Gewichtsprozent-Angaben auf das Gesamttrockengewicht der Partikel bezogen sind.

5. Duft- und/oder Aromaöl-enthaltender Partikel nach Anspruch 4 mit:
(i) einer mittleren Partikelgröße im Bereich von 5 bis 125 Mikronen, und/oder
(ii) einem Rest-Wassergehalt im Bereich von 0.1 bis 2.5 Gew.-%, und/oder
(iii) einer Menge an Oberflächenöl von weniger als 3 Gew.-%,
wobei alle Gewichtsprozent-Angaben auf das Gesamttrockengewicht der Partikel bezogen sind.

6. Verfahren zum Herstellen der Duft- und/oder Aromaöl-enthaltenden Partikel nach einem der Ansprüche 1 bis 5 bestehend aus:
i. Herstellen einer Mischung bestehend aus den folgenden Bestandteilen
(a) bis (e)
(a) 20 - 56 Gew.-% Duft- und/oder Aromaöl,
(b) 2 bis 17.5 Gew.-% modifizierte Stärke, wobei die modifizierte Stärke octenyl-succinylierte Stärke ist,
(c) 2 bis 17.5 Gew.-% eines oder mehrerer Phosphatsalze ausgewählt aus monoalkalinen Metallphosphaten,
(d) 0 - 7 Gew.-% eines oder mehrerer zusätzlicher Inhaltsstoffe und
(e) 30 - 60 Gew.-% Wasser,
jeweils bezogen auf das Gesamtgewicht der Mischung und dann
ii. Trocknen der Mischung aus Schritt i) um die Duft- und/oder Aromaöl-enthaltenden Partikel zu erhalten, wobei die Partikel keine andere modifizierte Stärke enthalten.

7. Verfahren zum Herstellen der Duft- und/oder Aromaöl-enthaltenden Partikel nach Anspruch 6, wobei die Partikel keine andere modifizierte Stärke enthalten, wobei eine oder mehrere der folgenden Mengen an Bestandteilen (a) bis (e) in Schritt i) bereitgestellt werden:
(a) 27.5 - 52 Gew.-% Duft- und/oder Aromaöl und/oder
(b) 2.25 - 11.25 Gew.-% modifizierte Stärke, wobei die modifizierte Stärke octenyl-succinylierte Stärke ist, und/oder
(c) 2.25 - 11.25 Gew.-% eines oder mehrerer Phosphatsalze ausgewählt aus monoalkalinen Metallphosphaten, und/oder
(d) 0 - 6.5 Gew.-% eines oder mehrerer zusätzlicher Inhaltsstoffe, und/oder
(e) 35 - 55 Gew.-% Wasser,
jeweils bezogen auf das Gesamtgewicht der Mischung.

8. Verfahren zum Herstellen der Duft- und/oder Aromaöl-enthaltende Partikel nach Anspruch 6 oder 7, wobei der Trocknungsschritt ii) ein Sprüh-Trocknungsschritt ist.

9. Duft- und/oder Aroma-enthaltender Partikel erhältlich durch das Verfahren nach einem der Ansprüche 6 bis 8.

10. Parfümiertes und/oder aromatisiertes Konsumprodukt enthaltend einen oder mehrere Duft- und/oder Aromaöl-enthaltende Partikel nach einem der Ansprüche 1 bis 5.

11. Verfahren zum Parfümieren oder Aromatisieren eines Konsumprodukts durch Zusatz eines oder mehrerer Duft- und/oder Aromaöl-enthaltenden Partikel nach einem der Ansprüche 1 bis 5 zu dem Konsumprodukt.

12. Verwendung eines oder mehrerer Duft- und/oder Aromaöl-enthaltender Partikel nach einem der Ansprüche 1 bis 5 zum Parfümieren oder Aromatisieren eines Konsumprodukts.

## Revendications

1. Particule contenant de l'huile parfumée et/ou aromatique, se composant de:
(a) 65 à 80 % en poids d'huile parfumée et/ou aromatique,
(b) 5 à 25 % en poids d'amidon modifié, l'amidon modifié étant de l'amidon octénylsuccinylé,
(c) 5 à 25 % en poids d'un ou de plusieurs sel(s) de phosphate choisi(s) parmi les phosphates de métal mono-alcalin,
(d) 0 à 10 % en poids d'un ou de plusieurs ingrédient(s) supplémentaire(s),
les pourcentages en poids se rapportant au poids total sec de la particule et les particules ne contenant pas d'autre amidon modifié.

2. Particule contenant de l'huile parfumée et/ou aromatique, selon la revendication 1, se composant de:
(a) 65 à 78 % en poids d'huile parfumée et/ou aromatique, et/ou
(b) de 10 à 25 % en poids d'amidon chimiquement modifié, l'amidon modifié étant de l'amidon octénylsuccinylé, et/ou
(c) de 5 à 15 % en poids de phosphate monosodique et/ou monopotassique, et/ou
(d) de 0 à 5 % en poids d'un ou de plusieurs ingrédient(s) supplémentaire(s),
les pourcentages en poids se rapportant au poids total sec de la particule et les particules ne contenant pas d'autre amidon modifié.

3. Particule contenant de l'huile parfumée et/ou aromatique, selon la revendication 2, se composant de:
(a) 65 à 75 % en poids d'huile parfumée ou aromatique,
(b) 15 à 22 % en poids d'amidon chimiquement modifié, l'amidon modifié étant de l'amidon octénylsuccinylé,
(c) 8 à 12 % en poids de phosphate monosodique et
(d) de 0 à 5 % en poids d'ingrédients supplémentaires,
et les particules ne contenant pas d'autre amidon modifié.

4. Particule contenant de l'huile parfumée et/ou aromatique, selon l'une quelconque des revendications 1 à 3, présentant:
(i) une taille de particule égale ou inférieure à 300 microns, et/ou
(ii) une teneur résiduelle en eau égale ou inférieure à 3 % en poids, et/ou
(iii) une quantité d'huile de surface égale ou inférieure à 4 % en poids,
l'ensemble des pourcentages en poids se rapportant au poids total sec des particules.

5. Particule contenant de l'huile parfumée et/ou aromatique, selon la revendication 4, présentant:
(i) une taille moyenne de particule comprise entre 5 et 125 microns et/ou
(ii) une teneur résiduelle en eau comprise entre 0.1 et 2.5 % en poids et/ou
(iii) une quantité d'huile de surface inférieure à 3 % en poids,
l'ensemble des pourcentages en poids se rapportant au poids total sec des particules.

6. Procédé de production des particules contenant de l'huile parfumée et/ou aromatique, selon l'une quelconque des revendications 1 à 5, consistant à:
i. préparer un mélange se composant des composants suivants (a) à (e)
(a) de 20 à 56 % en poids d'huile parfumée et/ou aromatique,
(b) de 2 bis 17.5 % en poids d'amidon modifié, l'amidon modifié étant de l'amidon octénylsuccinylé,
(c) de 2 bis 17.5 % en poids d'un ou de plusieurs sel(s) de phosphate choisi(s) parmi les phosphates de métal mono-alcalin,
(d) de 0 à 7 % en poids d'un ou de plusieurs ingrédients supplémentaires et
(e) de 30 à 60 % en poids d'eau,
respectivement par rapport au poids total du mélange, et, ensuite,
ii. à sécher le mélange de l'étape i) afin d'obtenir les particules contenant de l'huile parfumée et/ou aromatique, les particules ne contenant pas d'autre amidon modifié.

7. Procédé de production des particules contenant de l'huile parfumée et/ou aromatique, selon la revendication 6, lesdites particules ne contenant pas d'autre amidon modifié, l'une ou plusieurs des quantités suivantes de composants (a) à (e) étant fournie(s) à l'étape i):
(a) de 27.5 à 52 % en poids d'huile parfumée et/ou aromatique et/ou
(b) de 2.25 à 11.25 % en poids d'amidon modifié, l'amidon modifié étant de l'amidon octénylsuccinylé, et/ou
(c) de 2.25 à 11.25 % en poids d'un ou de plusieurs sel(s) de phosphate choisi(s) parmi les phosphates de métal mono-alcalin, et/ou
(d) de 0 à 6.5 % en poids d'un ou de plusieurs ingrédients supplémentaires et/ou
(e) de 35 à 55 % en poids d'eau,
respectivement par rapport au poids total du mélange.

8. Procédé de production des particules contenant de l'huile parfumée et/ou aromatique, selon la revendication 6 ou 7, dans lequel ladite étape de séchage ii) est une étape de séchage par pulvérisation.

9. Particule contenant du parfum et/ou de l'arôme, apte à être obtenue par le procédé selon l'une quelconque des revendications 6 à 8.

10. Produit de consommation parfumé et/ou aromatisé comprenant une ou plusieurs particule(s) contenant de l'huile parfumée et/ou aromatique, selon l'une quelconque des revendications 1 à 5.

11. Procédé destiné à parfumer ou à aromatiser un produit de consommation en ajoutant audit produit de consommation une ou plusieurs particule(s) contenant de l'huile parfumée et/ou aromatique, selon l'une quelconque des revendications 1 à 5.

12. Utilisation d'une ou de plusieurs particule(s) contenant de l'huile parfumée et/ou aromatique, selon l'une quelconque des revendications 1 à 5, pour parfumer ou aromatiser un produit de consommation.
